# EUROPÄISCHE PATENTSCHRIFT

(11) **EP 3 641 708 B1**
(45) Veröffentlichungstag und Bekanntmachung des Hinweises auf die Patenterteilung: **28.01.2026**
(21) Anmeldenummer: 18736825.3
(22) Anmeldetag: 21.06.2018
(51) Int. Cl.: A61F 5/56, A61C 7/36, A61C 7/08

(54) **OKKLUSIONSSCHIENENANORDNUNG MIT FIXIERBAND**
OCCLUSION SPLINT ARRANGEMENT WITH FIXING BAND
DISPOSITIF DE GOUTTIÈRE D'OCCLUSION AVEC UNE BANDE DE FIXATION

(30) Priorität: 22.06.2017 DE 102017113833
(43) Veröffentlichungstag der Anmeldung: 29.04.2020
(73) Patentinhaber: Hofmann, Konrad, 97291 Thüngersheim (DE)
(72) Erfinder: Hofmann, Konrad, 97291 Thüngersheim (DE)
(74) Vertreter: Stöckeler, Ferdinand
(86) Internationale Anmeldenummer: PCT/EP2018/066653
(87) Internationale Veröffentlichungsnummer: WO 2018/234498

(56) Entgegenhaltungen:
- EP-A1- 2 143 397
- CH-A1- 707 232
- CN-U- 203 815 696
- FR-A1- 2 964 853
- KR-B1- 101 482 728
- US-A- 5 755 219
- US-A1- 2015 216 716

## Beschreibung

Die Erfindung betrifft eine Okklusionsschienenanordnung, insbesondere zur Schlafapnoe-Therapie, mit einer maxillaren und einer mandibularen Miniplastschiene gemäß dem Oberbegriff des Patentanspruchs 1.

Insbesondere zur Schlafapnoe-Therapie sind aus dem Stand der Technik verschiedenste Ausführungsformen von Okklusionsschienenanordnungen bekannt. Diese zielen grundlegend darauf ab, die Stellung des Oberkiefers relativ zum Unterkiefer dahingehend zu beeinflussen, dass im tiefen Schlaf ein Zurücksacken des Unterkiefers sowie des Zungenmuskels und des Gaumensegels auf ein zulässiges Maß begrenzt wird, durch Verschiebung des Unterkiefers nach vorne (in X-Richtung), um das Gaumensegel so weit zu spannen, dass die Atemwege offengehalten werden und Schlafstörungen durch mangelnde Versorgung mit Atemluft reduziert bzw. gänzlich beseitigt werden.

Eine gattungsgemäße Okklusionsschienenanordnung ist aus der DE 103 41 260 A1 bekannt. Diese Okklusionsschienenanordnung weist eine maxillare Miniplastschiene und eine mandibulare Miniplastschiene auf, die jeweils auf den entsprechenden Zahnreihen im Oberkiefer bzw. im Unterkiefer aufgesteckt werden können. Weiterhin weisen diese Miniplastschienen einander gegenüberliegende Anlageflächen auf, die in einer Transversalebene labial in X-Richtung und bukkal in Y-Richtung relativ gegeneinander verschoben werden können. Zur Realisierung der Definition einer bestimmten Stellung der mandibularen Miniplastschiene relativ zur maxillaren Miniplastschiene weist die Okklusionsschieneneinrichtung eine Verstelleinrichtung mit einem Raststift und einer Rastführung auf. Durch Eingriff des Raststifts in die Rastführung wird die Position der mandibularen Miniplastschiene relativ zur maxillaren Miniplastschiene festgelegt. Allerdings hat diese Okklusionsschienenanordnung den Nachteil, dass nach Einrasten des Raststifts keinerlei Relativbewegung mehr zwischen den beiden Miniplastschienen möglich ist, was den Tragekomfort erheblich vermindert. Weiterhin nachteilig an dieser bekannten Okklusionsschienenanordnung sind deren komplexer Aufbau und die damit verbundenen hohen Herstellungskosten. Außerdem ist die Okklusionsschieneneinrichtung sehr anfällig gegen Schmutzablagerungen an schwer zu reinigenden Stellen, so dass die notwendige Hygiene nur schwierig zu gewährleisten ist.

CH 707232 A1 offenbart eine intraorale Schnarchtherapievorrichtung mit Ober- und Unterkieferschiene zur Stabilisierung des Unterkiefers des Patienten. Ein Fixierband wird hier in einem Tunnel der Oberkieferschiene geführt.

Aufgabe der vorliegenden Erfindung ist es daher, eine neue Okklusionsschienenanordnung vorzuschlagen, mittels der eine Zuordnung zwischen der mandibularen und der maxillaren Miniplastschiene ermöglicht wird und die dabei die Nachteile der aus dem Stand der Technik bekannten Okklusionsschienenanordnungen vermeidet.

Diese Aufgabe wird durch eine Okklusionsschienenanordnung nach der Lehre des Anspruchs 1 gelöst.

Vorteilhafte Ausführungsformen der Erfindung sind Gegenstand der Unteransprüche.

Die Okklusionsschienenanordnung dient vorzugsweise zur Schlafapnoe-Therapie. Wenngleich somit die vorliegende Ausführungsform einer Okklusionsschienenanordnung primär zur Therapierung des Schlafapnoe-Syndroms vorgesehen ist, ist es im Grundsatz unbenommen, die erfindungsgemäße Okklusionsschienenanordnung auch anderweitig einzusetzen. Hierbei kann die Okklusionsschienenanordnung beispielsweise auch zur Behandlung von Gebissfehlstellungen oder als Aufbissschiene zur Verhinderung eines nächtlichen Knirschens vorgesehen sein. Die erfindungsgemäße Okklusionsschienenanordnung wird durch das Positionierungsmittel zur Definition der Stellung der mandibularen Miniplastschiene relativ zur maxillaren Miniplastschiene in X-Richtung charakterisiert. Der Begriff Definition der Stellung ist dabei mit weitem Begriffsumfang zu verstehen, dahingehend dass nicht etwa eine ganz bestimmte Stellung definiert wird, sondern eine Stellung der beiden Miniplastschienen relativ zueinander, durch die der gewünschte Effekt erzielbar ist. Eine bestimmte Restbeweglichkeit für die beiden Zahnreihen im Oberkiefer und Unterkiefer relativ zueinander erhöht nämlich das Gefühl des subjektiven Tragekomforts erheblich. Bei der erfindungsgemäßen Okklusionsschienenanordnung dient als Positionierungsmittel ein Fixierband, mit dem Zugkräfte übertragen werden können. Die beiden Enden des Fixierbandes werden dabei an zwei Befestigungselementen fixiert, die im Backenzahnbereich der mandibularen Miniplastschiene angeordnet sind. Zugleich kann ein Mittelabschnitt des Fixierbandes, der sich üblicherweise auf der Mitte des Fixierbandes zu den Enden hin erstreckt, an einer Führungseinrichtung geführt werden, die im Schneidezahnbereich der maxillaren Miniplastschiene angeordnet ist. Durch die Führung des Mittelabschnitts des Fixierbandes im Schneidezahnbereich der maxillaren Miniplastschiene können von dem Mittelabschnitt Drucckräfte auf die labial gerichtete Außenseite der maxillaren Miniplastschiene übertragen werden. Zugleich werden die beiden Enden des Fixierbandes im Backenzahnbereich der mandibularen Miniplastschiene befestigt, so dass auch die Enden des Fixierbandes Zugkräfte auf die beiden Befestigungselemente an der mandibularen Miniplastschiene übertragen können. Im Ergebnis ist es damit möglich, abhängig von der Länge des Fixierbandes die mandibulare Miniplastschiene gegenüber der maxillaren Miniplastschiene nach vorne zu verspannen. Je kürzer das Fixierband gewählt wird, desto weiter wird die mandibulare Miniplastschiene zusammen mit dem Unterkiefer relativ zur maxillaren Miniplastschiene und dem Oberkiefer nach vorne gezogen.

Vorteil der erfindungsgemäßen Okklusionsschienenanordnung ist es zum einen, dass sich ein sehr einfacher Aufbau ergibt, der preisgünstig hergestellt werden kann. Außerdem bleibt dem Träger der Okklusionsschienenanordnung nach Anlegen der Miniplastschienen ein großer Bereich der Restbeweglichkeit zwischen den beiden Kieferhälften, da lediglich das Zurückfallen des Unterkiefers in Richtung des Gaumens durch die Länge des Fixierbandes begrenzt wird. Bewegungen der beiden Miniplastschienen relativ zueinander in seitlicher Richtung (Y-Richtung) werden durch das Fixierband dagegen nicht starr begrenzt, so dass der Träger der Okklusionsschienenanordnung einen hohen Tragekomfort empfindet.

In welcher Weise die Befestigungselemente bzw. die Führungseinrichtung an der mandibularen Miniplastschiene bzw. an der maxillaren Miniplastschiene befestigt sind, ist grundsätzlich beliebig. Da im Hinblick auf die Verträglichkeit verschiedener Materialien, die zur Herstellung der Okklusionsschienenanordnung verwendet werden, sehr hohe Anforderungen gestellt werden, ist es besonders vorteilhaft, wenn die beiden Befestigungselemente einstückig an die mandibulare Miniplastschiene angeformt oder eingeformt sind und/oder die Führungseinrichtung einstückig an die maxillare Miniplastschiene angeformt oder eingeformt ist. Durch die einstückige An- oder Einformung der Befestigungselemente bzw. der Führungseinrichtung an die jeweils zugehörigen Miniplastschienen kann für die Herstellung der Befestigungselemente bzw. der Erfindungseinrichtung das gleiche Material verwendet werden wie für die Herstellung der beiden Miniplastschienen selbst. Die einstückige Anformung oder Einformung erlaubt außerdem eine hohe mechanische Stabilität der Befestigungselemente bzw. der Führungseinrichtung, was im Hinblick auf ein unerwünschtes Lösen der Befestigungselemente bzw. der Führungseinrichtung von hoher Bedeutung ist.

Aus welchem Material die beiden Befestigungselemente bzw. die Führungseinrichtung hergestellt sind, ist grundsätzlich beliebig. Besonders vorteilhaft ist es, wenn die Befestigungselemente aus dem gleichen Kunststoffmaterial wie die mandibularen Miniplastschienen gebildet sind bzw. die Führungseinrichtung aus dem gleichen Kunststoffmaterial wie die maxillare Miniplastschiene gebildet ist. Durch die Verwendung des gleichen Kunststoffs sowohl für die beiden Miniplastschienen selbst als auch für die Befestigungselemente und/oder die Führungseinrichtung können Verträglichkeitsprobleme durch die zusätzliche Anordnung der Befestigungselemente und der Führungseinrichtung an den Miniplastschienen problemlos ausgeschlossen werden.

Mit welchem Herstellungsverfahren die erfindungsgemäßen Okklusionsschienenanordnungen hergestellt werden, ist grundsätzlich beliebig. So kann zur Herstellung der Okklusionsschienenanordnung ein Abdruck des Gebisses angefertigt werden, und auf dem daraus abgeleiteten Modell können die beiden Miniplastschienen urgeformt werden. Da die erfindungsgemäße Okklusionsschienenanordnung mit den beiden Befestigungselementen an der mandibularen Miniplastschiene und der Führungseinrichtung an der maxillaren Miniplastschiene einen sehr einfachen Aufbau aufweist, ist es aber möglich und grundsätzlich sehr vorteilhaft, dass die mandibulare Miniplastschiene zusammen mit den beiden einstückig angeformten oder eingeformten Befestigungselementen in einem 3D-Kunststoffdruckverfahren hergestellt ist und/oder dass die maxillare Miniplastschiene zusammen mit der einstückig angeformten oder eingeformten Führungseinrichtung in einem 3D-Kunststoffdruckverfahren hergestellt ist. Durch die Herstellung der beiden Miniplastschienen im 3D-Kunststoffdruckverfahren können Abformungsfehler, wie sie bei der Herstellung eines Abdrucks und der anschließenden Herstellung eines Modells und der anschließenden Urformung der Miniplastschienen auf den Modellen beinahe unvermeidlich sind, grundsätzlich ausgeschlossen werden. Stattdessen werden die beiden Kieferreihen mit entsprechenden Scannern abgescannt und die so erhobenen Geometriedaten zur Steuerung des 3D-Kunststoffdruckvefahrens mit bekannten CAD/CAM-Verfahren genutzt. Außerdem kann die Herstellung der Okklusionsschienenanordnung durch Verwendung von 3D-Kunststoffdruckern stark rationalisiert werden.

Alternativ zur Herstellung im 3D-Kunststoffdruck ist die Herstellung der Okklusionsschienenanordnung auch durch Verwendung von CADgesteuerten Mehrachsfräsverfahren möglich.

Die Definition der Stellung der mandibularen Miniplastschiene relativ zur maxillaren Miniplastschiene in X-Richtung durch das Fixierband wird maßgeblich durch die Länge des Fixierbandes vorgegeben. Je kürzer das Fixierband ist, desto weiter wird die mandibulare Miniplastschiene mit dem daran gehaltenen Unterkiefer nach vorne gezogen und dadurch das Gaumensegel gespannt. Um die Stellung der beiden Miniplastschienen relativ zueinander in einfacher Weise anpassen zu können, ist es besonders vorteilhaft, wenn das Fixierband längenverstellbar ist. Durch Verlängern bzw. Verkürzen des Fixierbandes kann dann die Stellung der beiden Miniplastschienen relativ zueinander individuell auf die Bedürfnisse des jeweiligen Trägers angepasst werden. Alternativ dazu können der Okklusionsschienenanordnung auch mehrere Fixierbänder mit unterschiedlichen Längen beigelegt werden, um dem Benutzer durch Auswahl eines bestimmten Fixierbandes die entsprechende Anpassung der damit erreichbaren Relativstellung der beiden Miniplastschienen zueinander zu ermöglichen.

Aus welchem Material das Fixierband hergestellt ist, ist grundsätzlich beliebig. Um Verträglichkeitsprobleme beim Tragen der Okklusionsschienenanordnung durch das Material des Fixierbandes möglichst einfach auszuschließen, ist es besonders vorteilhaft, wenn das Fixierband aus Kunststoff hergestellt wird, da mundhöhlenverträgliche Kunststoffe problemlos verfügbar sind.

Im Hinblick auf eine reproduzierbare und definierte Wirkung der Okklusionsschienenanordnung bei der Schlafapnoe-Therapie ist es von großer Bedeutung, dass das Gaumensegel eine bestimmte Mindestspannung durchgängig aufweist. Um dies jederzeit zu gewährleisten, ist es besonders vorteilhaft, dass das Fixierband in Richtung seiner Längsachse hochsteif ausgebildet ist und im Wesentlichen keine elastische Verformung bei den im bestimmungsgemäßen Gebrauch auftretenden Belastungen zulässt. Dadurch wird ausgeschlossen, dass auch bei unwillkürlichen Bewegungen der beiden Kieferreihen gegeneinander der Unterkiefer aufgrund einer unerwünschten Elastizität des Fixierbandes relativ zu dem Oberkiefer so weit zugezogen wird, dass das Gaumensegel die gewünschte Spannung unterschreitet.

Gemäß einer bevorzugten Ausführungsform ist es vorgesehen, dass das Fixierband aus einem formstabilen, elastisch verformbaren Kunststoff hergestellt ist. Das Fixierband weist dabei im belastungsfreien Zustand eine bogenförmige Gestalt auf. Im Ergebnis hat das vorgeformte Fixierband also eine Form, die ungefähr der Form entspricht, die das Fixierband nach der Fixierung an den Miniplastschienen einnimmt. Durch diese der Einbauform entsprechende Vorformung wird die Materialspannung im Material des Fixierbandes deutlich reduziert, so dass das Fixierbandes zur Fixierung an den Miniplastschienen durch den Benutzer gar nicht oder nur minimal verformt werden muss.

Für die konstruktive Ausbildung der Führungseinrichtung bzw. der Befestigungselemente an der maxillaren Miniplastschiene bzw. der mandibularen Miniplastschiene gibt es eine Vielzahl von Möglichkeiten. Es ist vorgesehen, dass die Führungseinrichtung einer mandibularen Miniplastschiene in der Art einer Nut ausgebildet ist. Diese Nut verläuft dann im Schneidezahnbereich der maxillaren Miniplastschiene labial, d.h. an der Außenseite der Miniplastschiene und parallel zum Zahnbogen. In diese Nut der maxillaren Miniplastschiene kann das Fixierband von außen eingelegt werden, so dass das Fixierband Druckkräfte auf den Nutgrund übertragen kann. Mittels dieser von dem Mittelabschnitt des Fixierbandes auf den Nutgrund der Nut übertragenen Druckkräfte kann dann die notwendige Zugspannung im Fixierband aufgebaut werden, mit der die mandibulare Miniplastschiene gegenüber der maxillaren Miniplastschiene nach vorne gezogen und in ihrer Position definiert wird.

Soll das Fixierband beispielsweise mit einem Presssitz formschlüssig in der Nut der Miniplastschiene fixiert werden, so ist es vorteilhaft, wenn das Fixierband quer zu seiner Längsachse elastisch verformbar ausgebildet ist. Durch diese elastische Verformbarbarkeit des Fixierbandes kann das Fixierband zumindest geringfügig quer zu seiner Längsachse zusammengedrückt und durch eine verengte Nutöffnung in die Nut hinein gedrückt werden.

Die Länge der Nut in der maxillaren Miniplastschiene sollte so groß sein, dass eine ausreichende Führung des Fixierbandes an der maxillaren Miniplastschiene jederzeit gewährleistet ist. Zugleich sollte gemäß einer bevorzugten Ausführungsform zwischen den beiden Enden der Nut in der maxillaren Miniplastschiene und den beiden Befestigungselementen an der mandibularen Miniplastschiene jeweils ein Abstand vorhanden sein, in dem das Fixierband mit einem freien Längenabschnitt ungeführt verläuft. Durch diesen freien Längenabschnitt des Fixierbandes wird gewährleistet, dass eine Verschiebung der mandibularen Miniplastschiene relativ zur maxillaren Miniplastschiene zumindest in Y-Richtung, d.h. quer zur Spannrichtung, ermöglicht wird.

Um den Öffnungswinkel des Unterkiefers relativ zum Oberkiefer durch die Okklusionsschienenanordnung kontrollieren zu können, ist es vorteilhaft, wenn zwischen den beiden seitlichen Enden der Nut in der maxillaren Miniplastschiene und den beiden Befestigungselementen an der mandibularen Miniplastschiene jeweils ein Abstand vorhanden ist, wobei an der mandibularen Miniplastschiene ein rechtes und ein linkes Umlenkelement vorgesehen sind, an denen das Fixierband umgelenkt wird und dadurch die vertikale Relativbewegung zwischen der maxillaren Miniplastschiene und der mandibularen Miniplastschiene begrenzt wird. Im Ergebnis kann durch die Umlenkelemente bestimmt werden, wie stark der Unterkiefer gegen den Oberkiefer gezogen wird.

Gemäß einer bevorzugten Ausführungsform ist es vorgesehen, dass an der Öffnung der Nut eine oder mehrere Verengungen vorgesehen sind, durch die das, insbesondere im Querschnitt kreisrunde, Fixierband formschlüssig in der Nut fixiert wird. Im Ergebnis kann somit der Sitz des Fixierbandes in der Nut gesichert werden. Dies ist insbesondere vorteilhaft, wenn der Benutzer zunächst die beiden nicht miteinander verbundenen Miniplastschienen an Oberkiefer und Unterkiefer anlegt und erst danach die beiden Miniplastschienen durch Fixierung des Fixierbandes in der Nut miteinander verbindet.

Weiterhin besonders vorteilhaft ist es, wenn an der nach außen weisenden Seite der Verengungen eine Einführschräge vorgesehen ist, durch die das Fixierband in die Nut geleitet wird. Dies ist insbesondere vorteilhaft, wenn der Benutzer zunächst die beiden nicht miteinander verbundenen Miniplastschienen an Oberkiefer und Unterkiefer anlegt und erst danach die beiden Miniplastschienen durch Fixierung des Fixierbandes in der Nut miteinander verbindet.

Für die Funktion der erfindungsgemäßen Okklusionsschienenanordnung insbesondere bei der Schlafapnoe-Therapie ist es von großer Bedeutung, dass sich das Fixierband nicht ungewollt, beispielsweise während des Schlafs, aus der Nut an der maxillaren Miniplastschiene löst, da ansonsten die bestimmungsgemäße Fixierung der mandibularen Miniplastschiene relativ zur maxillaren Miniplastschiene nicht mehr gewährleistet ist. Um diese Sicherung des Fixierbandes in der Nut zu gewährleisten, können an der Öffnung der Nut Fixierzapfen und/oder Fixierstäbe vorgesehen sein, die die Öffnung der Nut abschnittsweise oder entlang der vollen Länge der Nut verengen. Beim Einführen des Fixierbandes in die Nut hat der Benutzer dann die Möglichkeit, das Fixierband beispielsweise geringfügig elastisch zu verformen und dadurch durch die verengte Öffnung der Nut hineinzudrücken. Ein unwillkürliches Lösen des Fixierbandes aus der durch die Fixierzapfen bzw. die Fixierstege verengten Nutöffnung ist ausgeschlossen. Soweit zur Sicherung des Fixierbandes und der Nut Fixierzapfen eingesetzt werden, ist es besonders vorteilhaft, wenn zumindest drei Fixierzapfen vorgesehen sind. Jeweils benachbarte Fixerzapfen werden dann abwechselnd an den einander gegenüberliegenden Seitenkanten der Nut angeordnet und kragen somit mit ihren freien Enden jeweils in Gegenrichtung in die Öffnung. Zum Einführen des Fixierbandes in die so ausgestaltete Nut mit den mehreren Fixierzapfen kann das zunächst noch ungespannte Fixierband in die Nut eingeführt werden, wobei das Fixierband beim Passieren der jeweils benachbarten Fixierzapfen abwechselnd nach oben bzw. unten gebogen wird.

Alternativ zur Verwendung von Fixerzapfen können an der Öffnung der Nut zur Sicherung des Fixierbandes auch Fixierstege vorgesehen sein. Diese Fixierstege können sich gemäß einer bevorzugten Ausführungsform durchgehend zwischen den beiden seitlichen Enden der Nut erstrecken. Der Öffnungsquerschnitt zwischen den beiden Fixierstegen sollte dann zumindest so groß sein, dass bei zulässiger Verformung des Querschnitts des Fixierbandes ein Einführen des Fixierbandes in die Nut ermöglicht wird.

Gemäß einer bevorzugten Ausführungsform ist es vorgesehen, dass der Querschnitt der Nut zumindest geringfügig kleiner als der Querschnitt des Fixierbandes ist, wobei das Fixierband durch den dadurch gebildeten Presssitz in der Nut fixiert wird. Der Presssitz bewirkt aufgrund der Reibung zwischen Nutoberfläche und Fixierband, dass sich das Fixierband nach dem Einpressen in den Nutgrund nicht mehr verschieben kann.

Besonders effektiv kann ein Presssitz realisiert werden, wenn das Fixierband einen kreisförmigen Querschnitt aufweist.

Bei Bewegungen der mandibularen Miniplastschiene relativ zur maxillaren Miniplastschiene in Y-Richtung, d.h. quer zur Spannrichtung, werden die beiden Befestigungselemente an der mandibularen Miniplastschiene in Y-Richtung relativ zur maxillaren Miniplastschiene verschoben. Um diese Bewegung nicht unerwünscht einzuschränken, ist es besonders vorteilhaft, wenn der Querschnitt der Nut größer als der Querschnitt des Fixierbandes ist, so dass kein wesentlicher Reibschluss zwischen dem Fixierband und der Nutoberfläche auftritt. Auf diese Weise kann dann das Fixierband entlang seiner Längsachse im Wesentlichen widerstandsfrei in der Nut verschoben werden, so dass die Bewegung der mandibularen Miniplastschiene in Y-Richtung durch die reibschlüssige Fixierung des Fixierbandes in der Nut nicht behindert wird. Die erfindungsgemäße Spannwirkung des Fixierbandes auf die mandibulare Miniplastschiene wird dadurch nicht negativ beeinflusst, da für diese Spannwirkung lediglich die Übertragung von Druckkräften von dem Fixierband auf den Nutgrund der Nut erforderlich ist. Zur Sicherung des Fixierbandes in der Nut ist es gemäß einer weiteren Variante auch möglich, dass das Fixierband einen rechteckförmigen Querschnitt aufweist, wobei die Breite des Fixierbandes größer als der Öffnungsquerschnitt der Nut ist, und wobei die Höhe des Fixierbandes kleiner als der Öffnungsquerschnitt der Nut ist. Beim Einführen des Fixierbandes in die Nut wird dann das Fixierband so gehalten, dass es mit seiner schmaleren Breitseite in den Öffnungsquerschnitt der Nut eingeführt wird. Sobald das Fixierband sich über die volle Länge der Nut erstreckt, kann das Fixierband dann um 90° gedreht werden, so dass ein Zurückziehen des Fixierbandes durch den Öffnungsquerschnitt aufgrund der größeren Breite des Fixierbandes ausgeschlossen ist. Die Lage des Fixierbandes in dieser Position kann dann durch Einhängen der Enden des Fixierbandes an den Befestigungseinrichtungen der mandibularen Miniplastschiene gesichert werden.

Bevorzugt sollte das Fixierband im Schneidezahnbereich mit der Längsseite des rechteckförmigen Querschnitts parallel zur Außenseite der Schneidezähne, das heißt parallel zum Zahnbogen, verlaufen. Denn damit ist gewährleistet, dass das Fixierband in Richtung der größten Querbelastung die höchste Steifigkeit aufweist.

In welcher Art die beiden Befestigungselemente an der mandibularen Miniplastschiene ausgebildet sind, ist grundsätzlich beliebig. Besonders einfach und kostengünstig ist die Realisation der Befestigungselemente möglich, wenn diese in der Art von Fixierzapfen ausgebildet sind, die im Backenzahnbereich der mandibularen Miniplastschiene seitlich überstehen. Die freie Länge dieser Fixierzapfen kann sehr kurz sein, da für das Fixieren der Enden des Fixierbandes nur ein relativ kleiner Überstand der Fixierzapfen erforderlich ist. Außerdem können solche Fixierzapfen sehr gut einstückig an dem Kunststoff der mandibularen Miniplastschiene angeformt, insbesondere im 3D-Kunststoffdruck angedruckt, werden. Die Geometrie der Fixierzapfen ist dabei so zu wählen, dass die Enden des Fixierbandes mit einer Ausnehmung oder einem Hakenelement an den Fixierzapfen fixierend eingehängt werden können.

Um ein unerwünschtes Lösen der Enden des Fixierbandes von den Fixierzapfen auszuschließen, ist es gemäß einer bevorzugten Ausführungsform vorgesehen, dass am freien Ende der Fixierzapfen ein Sicherungselement vorgesehen ist. Dieses Sicherungselement kann beispielsweise in der Art einer Verdickung ausgebildet sein, deren Querschnitt größer ist als der Querschnitt einer Ausnehmung am Ende des Fixierbandes, mit der das Fixierband am Fixierzapfen eingehängt wird. Das Material des Fixierbandes muss dann eine so große Verformbarkeit aufweisen, dass das Ende des Fixierbandes mit der darin vorgesehenen Ausnehmung unter Überwindung der Verdickung auf den Fixierzapfen aufgeschoben werden kann.

Bevorzugt ist es, wenn am freien Ende der Fixierzapfen jeweils zumindest ein Überstand vorgesehen ist, wobei in einer Montageposition des Fixierbandes der Überstand in eine Öffnung am Ende des Fixierbandes eingeführt werden kann, und wobei in einer Fixierposition des Fixierbandes der Überstand die Öffnung am Ende des Fixierbandes fixierend hintergreift. Im Ergebnis kann der Benutzer also durch entsprechendes Verdrehen der Enden des Fixierbandes dieses im noch nicht verbundenen Zustand problemlos an den Fixierzapfen einhängen. In der Nutzungsposition des Fixierbandes befinden sich die Enden des Fixierbandes dann in der Fixierposition, so dass ein ungewolltes Lösen der Enden des Fixierbandes von den Fixierzapfen durch den Hintergriff der Überstände zuverlässig ausgeschlossen ist.

Insbesondere bei vorgeformten Fixierbändern muss gewährleistet sein, dass das Fixierband entsprechend seiner Vorformung lagerichtig an den Fixierzapfen eingehängt wird. Um dies zu gewährleisten, kann das Sicherungselement am rechten Fixierzapfen eine andere Form als das Sicherungselement am linken Fixierzapfen aufweisen. Insbesondere kann die Verdickung am rechten Fixierzapfen einen anderen Durchmesser aufweisen als die Verdickung am linken Fixierzapfen. Die Öffnung am linken Ende des Fixierbandes ist dabei auf die Form des Sicherungselements am linken Fixierzapfen und die Öffnung am rechten Ende des Fixierbandes auf die Form des Sicherungselements am rechten Fixierzapfen abgestimmt, so dass der Benutzer die Enden des Fixierbandes dadurch nur lagerichtig an den Fixierzapfen einhängen kann.

Die seitlich an der mandibularen Miniplastschiene überstehenden Befestigungselemente können bei empfindlichen Verwendern zu Irritationen der Mundschleimhaut im Backenbereich führen. Um diesen Irritationen der Mundschleimhaut entgegenzuwirken, ist gemäß einer bevorzugten Variante vorgesehen, dass benachbart zu den Befestigungselementen an der mandibularen Miniplastschiene jeweils glattflächige Abdeckelemente vorgesehen sind, die entsprechend den Befestigungselementen über die Miniplastschiene überstehen und auf diese Weise die Befestigungselemente seitlich zumindest teilweise abdecken. Aufgrund ihrer Glattflächigkeit können die Abdeckelemente dabei die Irritation der Mundschleimhaut wesentlich verringern bzw. gänzlich ausschließen.

Um die Anpassung der Okklusionsschienenanordnung auf den individuellen Bedarf eines Patienten abstimmen zu können, ist es vorteilhaft, wenn auf den beiden Miniplastschienen Markierungen angebracht sind, die die Position der beiden Miniplastschienen relativ zueinander markieren. Dadurch wird insbesondere die Auswahl eines Fixierbandes mit geeigneter Länge erleichtert.

Verschiedene Ausführungsformen der Erfindung sind in den Zeichnungen schematisch dargestellt und werden nachfolgend beispielhaft erläutert.

Es zeigen:
- **Fig. 1**: eine Okklusionsschienenanordnung mit Fixierband zur Definition der Stellung zwischen mandibularer Miniplastschiene und maxillarer Miniplastschiene in perspektivischer Ansicht von oben;
- **Fig. 2**: die Okklusionsschienenanordnung gemäß Fig. 1 in seitlicher Ansicht;
- **Fig. 3**: die mandibulare Miniplastschiene der Okklusionsschienenanordnung gemäß Fig. 1 in perspektivischer Ansicht von oben;
- **Fig. 4**: das Befestigungselement der mandibularen Miniplastschiene gemäß Fig. 3 im Teilquerschnitt entlang der Schnittlinie I-I;
- **Fig. 5**: das Befestigungselement gemäß Fig. 4 nach Anbringung des Endes eines Fixierbandes;
- **Fig. 6**: eine zweite Ausführungsform eines Befestigungselements zur Anordnung an einer Miniplastschiene im Teilquerschnitt;
- **Fig. 7**: das Befestigungselement gemäß Fig. 6 nach Anbringung des Endes eines Fixierbandes;
- **Fig. 8**: die maxillare Miniplastschiene der Okklusionsschienenanordnung gemäß Fig. 2 im Teilquerschnitt entlang der Schnittlinie II-II;
- **Fig. 9**: eine zweite Ausführungsform einer Miniplastschiene im Teilquerschnitt;
- **Fig. 10**: eine dritte Ausführungsform einer Miniplastschiene im Teilquerschnitt;
- **Fig. 11**: die Miniplastschiene gemäß Fig. 10 in Ansicht von vorne;
- **Fig. 12**: eine zweite Ausführungsform einer Okklusionsschienenanordnung in seitlicher Ansicht;
- **Fig. 13**: eine zweite Ausführungsform eines Befestigungselements einer mandibularen Miniplastschiene mit einem Ende des zugehörigen Fixierbandes in Ansicht von oben;
- **Fig. 14**: eine dritte Ausführungsform des rechten und linken Befestigungselements einer mandibularen Miniplastschiene mit dem rechten und linken Ende des zugehörigen Fixierbandes in Ansicht von oben;
- **Fig. 15**: ein vorgeformtes Fixierband in Ansicht von oben;
- **Fig. 16**: das vorgeformte Fixierband gemäß Fig. 15 in seitlicher Ansicht;
- **Fig. 17**: ein mit einem Presssitz in einer Nut fixiertes Fixierband im Querschnitt;
- **Fig. 18**: das Fixierband gemäß Fig. 17 beim Einführen in die Nut gemäß Fig. 17 im Querschnitt;
- **Fig. 19**: eine dritte Ausführungsform einer Okklusionsschienenanordnung in seitlicher Ansicht;
- **Fig. 20**: die maxillare Miniplastschiene der Okklusionsschienenanord- nung gemäß Fig. 19 in seitlicher Ansicht;
- **Fig. 21**: die mandibulare Miniplastschiene der Okklusionsschienenanordnung gemäß Fig. 19 in Ansicht von oben;
- **Fig. 22**: das Befestigungselement der mandibularen Miniplastschiene gemäß Fig. 21 mit daran befestigtem Fixierband im Teilquerschnitt;
- **Fig. 23**: das vorgeformte Fixierband der Okklusionsschienenanordnung gemäß Fig. 19 in seitlicher perspektivischer Ansicht;
- **Fig. 24**: das vorgeformte Fixierband gemäß Fig. 23 in Ansicht von oben.

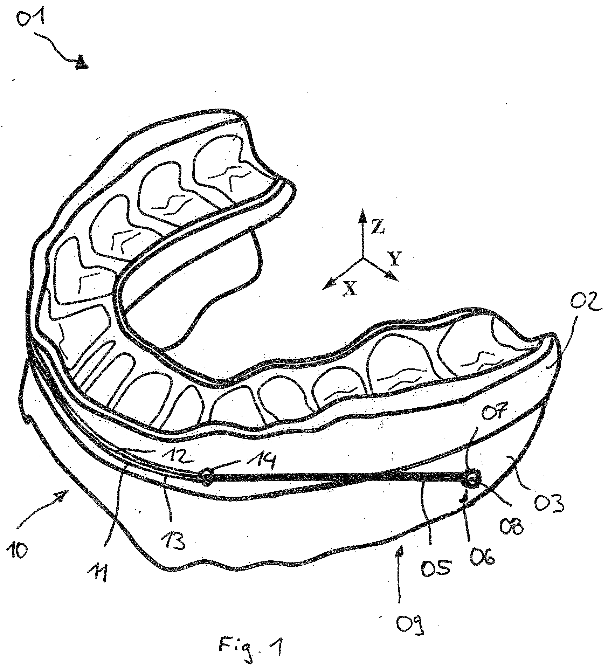

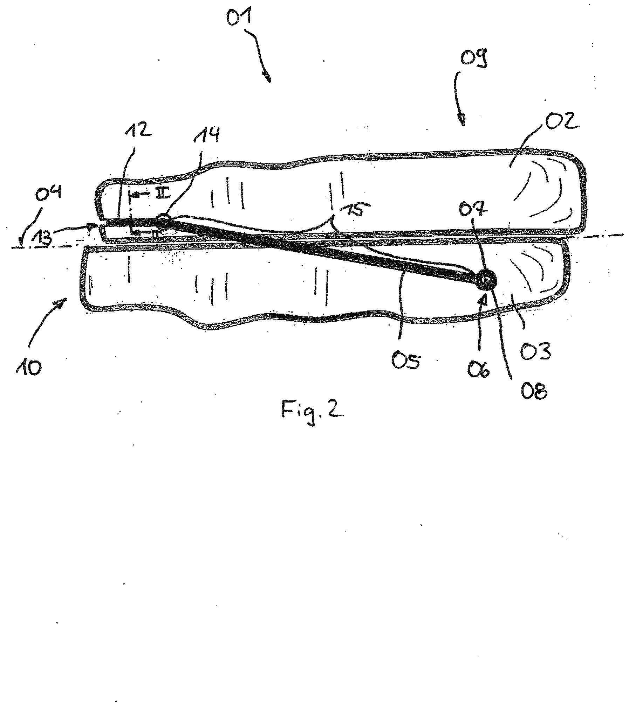

**Fig. 1** zeigt eine Okklusionsschienenanordnung 01, wie sie beispielsweise zur Schlafapnoe-Therapie eingesetzt werden kann. Die Okklusionsschienenanordnung 01 umfasst eine maxillare Miniplastschiene 02 zur Anordnung an der Zahnreihe des Oberkiefers und eine mandibulare Miniplastschiene 03 zur Anordnung an der Zahnreihe des Unterkiefers. Die beiden Miniplastschienen 02 und 03 sind in einer Transversalebene 04 (siehe Fig. 2) labial in X-Richtung und bukkal in Y-Richtung relativ gegeneinander verschiebbar.

Um im Rahmen der Schlafapnoe-Therapie eine Erhöhung der Spannung im Gaumensegel erreichen zu können, ist es erforderlich, dass die mandibulare Miniplastschiene 03 in X-Richtung relativ zur maxillaren Miniplastschiene 02 nach vorne gezogen und dort gehalten wird. Zur Definition der dabei einzuhaltenden Stellung zwischen der mandibularen Miniplastschiene 03 und der maxillaren Miniplastschiene 02 dient ein Fixierband 05. Die beiden Enden 06 des Fixierbandes 05 weisen jeweils ringförmige Ausnehmungen 07 auf und können an Befestigungselementen 08, die einstückig an die mandibulare Miniplastschiene 03 angeformt sind, eingehängt werden. In Fig. 1 ist lediglich das eine Ende 06 des Fixierbandes 05 dargestellt. Das zweite Ende 06 des Fixierbandes 05 wird in gleicher Weise auf der rechten Seite der mandibularen Miniplastschiene 03 an einem zweiten Befestigungselement 08 mit einer Ausnehmung 07 eingehängt. Beide Befestigungselemente 08 sind im Backenzahnbereich 09 der Miniplastschiene 03 angeordnet und stehen bukkal von der Seitenfläche der Miniplastschiene 03 ab.

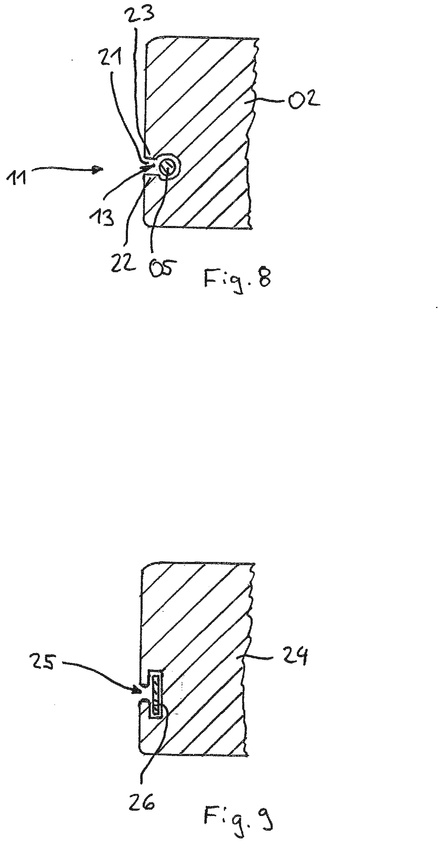

Die maxillare Miniplastschiene 02 weist im Schneidezahnbereich 10 eine Führungseinrichtung 11 auf, in der ein Mittelabschnitt 12 des Fixierbandes 05 geführt ist. Die Führungseinrichtung 11 ist in der Art einer Nut 13 ausgebildet (siehe Fig. 8) und erstreckt sich zwischen ihren Enden 14, von denen in Fig. 1 nur das linke Ende 14 dargestellt ist, durch das Kunststoffmaterial der maxillaren Miniplastschiene 02.

Sind die beiden Enden 06 des Fixierbandes 05 an den Befestigungselementen 08 der mandibularen Miniplastschiene 03 einhängt und verläuft der Mittelabschnitt 12 des Fixierbandes 05 durch die Nut 13, so kann abhängig von der Länge des Fixierbandes 05 die mandibulare Miniplastschiene 03 gegenüber der maxillaren Miniplastschiene 02 in X-Richtung nach vorne gespannt werden, um dadurch die gewünschte Spannung des Gaumensegels zu beeinflussen.

**Fig. 2** zeigt die Okklusionsschienenanordnung 01 mit den beiden Miniplastschienen 02 und 03 in seitlicher Ansicht. Das Fixierband 05 ist aus einem quer zu seiner Längsachse verformbaren, aber in Längsrichtung hochsteifen Kunststoff hergestellt, so dass sich das Fixierband 05 in Zugrichtung im Wesentlichen nicht elastisch dehnen lässt. Auf diese Weise wird ausgeschlossen, dass der Benutzer durch Aufbringung entsprechender Muskelkräfte die Miniplastschiene 03 unter elastischer Verformung des Fixierbandes 05 entgegen der Spannrichtung nach hinten zieht. Zugleich ist zwischen dem Ende 14 der Nut 13 einerseits und dem Befestigungselement andererseits ein freier Längenabschnitt 15 vorhanden, in dem das Fixierband auf der linken und rechten Seite der Okklusionsschienenanordnung 01 ungeführt zwischen den beiden Miniplastschienen 02 und 03 verläuft. Dieser freie Längenabschnitt 15 des Fixierbandes 05 ermöglicht eine Bewegung der beiden Miniplastschienen 02 und 03 in der Transversalebene quer zur X-Richtung, nämlich in Y-Richtung.

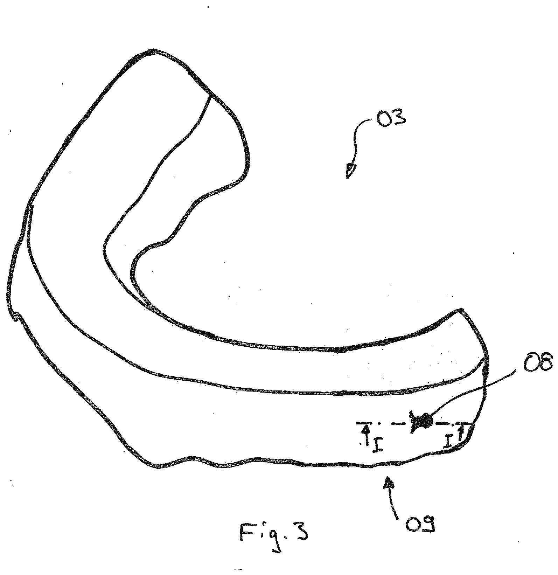

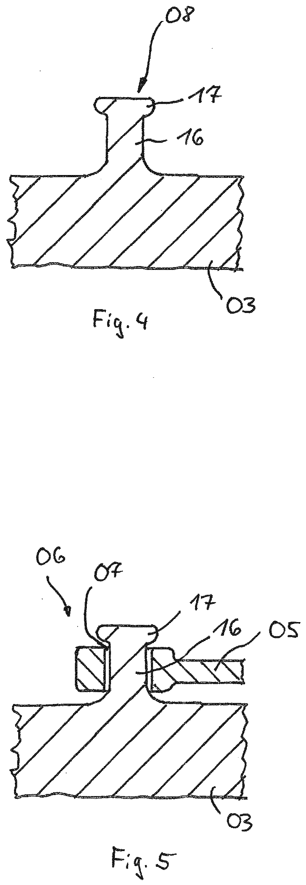

**Fig. 3** zeigt die mandibulare Miniplastschiene 03 mit dem im Backenzahnbereich 09 angebrachten Befestigungselement 08 in perspektivischer Ansicht. Das Befestigungselement 08 besteht aus dem gleichen Kunststoff wie die mandibulare Miniplastschiene 03 selbst. Der Aufbau des Befestigungselements 08 wird nachfolgend anhand der Zeichnungen Fig. 4 und Fig. 5 näher erläutert.

**Fig. 4** zeigt das Befestigungselement 08 der mandibularen Miniplastschiene 03 im Teilquerschnitt entlang der Schnittlinie I-I. Das Befestigungselement 08 ist in der Art eines Fixierzapfens 16 ausgebildet, der seitlich von der Miniplastschiene 03 übersteht. Der Fixierzapfen 16 und damit das Befestigungselement 08 ist dabei einstückig an die Miniplastschiene 03 angeformt und besteht aus dem gleichen Kunststoff wie die Miniplastschiene 03. Besonders einfach und kostengünstig kann die Miniplastschiene 03 mit dem Befestigungselement 08, nämlich dem Fixierzapfen 16, durch 3D-Kunststoffdruck hergestellt werden. Am freien Ende des Fixierzapfens 16 befindet sich ein Sicherungselement 17, das in der Art einer Verdickung des Fixierzapfens 16 ausgebildet ist.

**Fig. 5** zeigt den Fixierzapfen 16 nach Befestigung des Fixierbandes 05 durch Aufschieben der Ausnehmung 07 auf den Fixierzapfen 16. Ein ungewolltes Lösen des Fixierbandes 05 vom Fixierzapfen 16 wird durch das Sicherungselement 17, das einen größeren Querschnitt als die Ausnehmung 07 aufweist, verhindert.

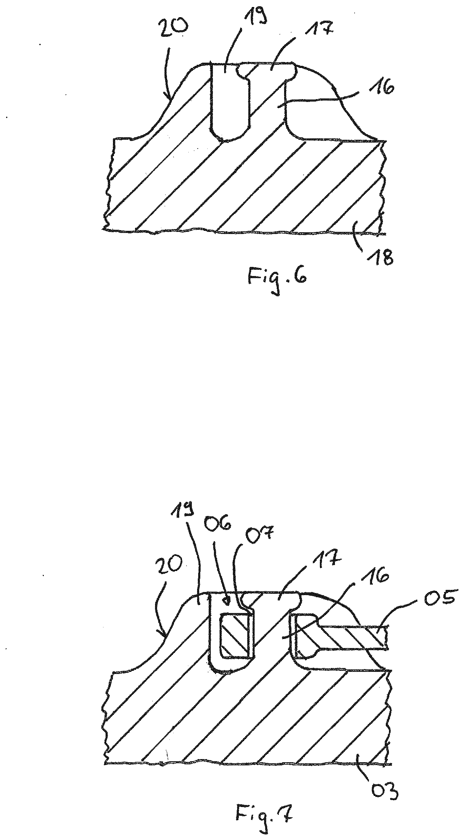

**Fig. 6** zeigt eine alternative Ausführungsform 18 einer Miniplastschiene, an der ebenfalls ein Fixierzapfen 16 einstückig angeformt ist. Im Unterschied zur Miniplastschiene 03 ist an der Miniplastschiene 18 ein Abdeckelement 19 einstückig angeformt, das benachbart zum Fixierzapfen 16 angeordnet ist. Das Abdeckelement 19 steht dabei mit der gleichen freien Länge über die Miniplastschiene 18 über wie der Fixierzapfen 16 und deckt den Fixierzapfen 16 mit einer glattflächigen Seitenfläche 20 ab. Durch die Abdeckung durch das Abdeckelement 19 können Irritationen der Schleimhaut im Bereich der Backen beim Tragen der mandibularen Miniplastschiene 18 vermieden werden.

**Fig. 7** zeigt die mandibulare Miniplastschiene 18 mit dem Fixierzapfen 16 und dem Abdeckelement 19 nach Anbringung des Fixierbandes 05 am Fixierzapfen 16.

**Fig. 8** zeigt die maxillare Miniplastschiene 02 im Teilquerschnitt entlang der Schnittlinie II-II (siehe Fig. 2). Die Führungseinrichtung 11 an der Miniplastschiene 02 wird durch die Nut 13 gebildet, die einstückig in den Kunststoff der maxillaren Miniplastschiene 02 eingeformt ist. Dies kann sehr einfach und kostengünstig durch 3D-Kunststoffdruck hergestellt werden. Der Querschnitt der Nut 13 ist etwas größer als der Querschnitt des Fixierbandes 05, so dass das Fixierband entlang seiner Längsachse im Wesentlichen widerstandsfrei in der Nut 13 verschoben werden kann, da im Wesentlichen kein Reibschluss zwischen dem Fixierband 05 und der Innenseite der Nut 13 vorhanden ist. An der Öffnung 21 der Nut 13 sind zwei Fixierstege 22 und 23 vorgesehen, die die Öffnung 21 der Nut 13 verengen. Der verbleibende Öffnungsquerschnitt der Öffnung 21 ist dabei geringfügig kleiner als der Querschnitt des Fixierbandes 05, so dass ein Herausrutschen des Fixierbandes 05 aus der Nut 13 während des Tragens der Okklusionsschienenanordnung 01 ausgeschlossen ist.

**Fig. 9** zeigt eine alternative Ausführungsform 24 einer maxillaren Miniplastschiene, deren Führungseinrichtung wiederum in der Art einer Nut 25 ausgebildet ist. In der Nut 25 ist ein rechteckiges Fixierband 26 geführt, wobei die Breite des Fixierbandes 26 größer ist als der Öffnungsquerschnitt der Nut 25, und wobei die Höhe des Fixierbandes 26 kleiner ist als der Öffnungsquerschnitt der Nut 25. Zum Einführen des Fixierbandes 26 wird dieses gegenüber der Darstellung in Fig. 9 um 90° gedreht und mit einer Seitenkante in die Nut 25 eingefädelt. In der Nut 25 wird das Fixierband 26 dann wiederum um 90° zurückgedreht und kann aufgrund der größeren Breite gegenüber dem Öffnungsquerschnitt der Nut 25 während des Tragens der maxillaren Miniplastschiene 24 nicht mehr aus der Nut 25 herausrutschen. Dadurch, dass das Fixierband im Schneidezahnbereich mit der Längsseite seines rechteckförmigen Querschnitts parallel zur Außenseite der Schneidezähne, das heißt parallel zum Zahnbogen verläuft, weist es eine hohe Steifigkeit in der Hauptbelastungsrichtung auf.

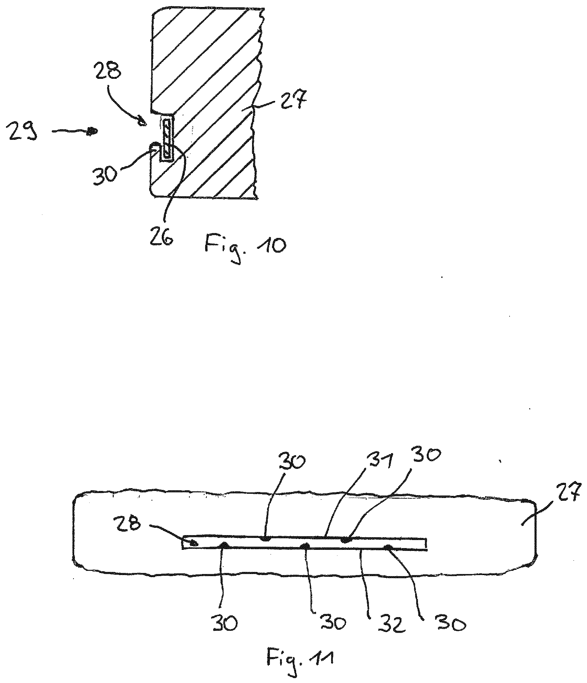

**Fig. 10** und **Fig. 11** zeigen eine weitere Ausführungsform 27 einer maxillaren Miniplastschiene, die wiederum eine Nut 28 als Führungseinrichtung 29 enthält. Zur Sicherung des Fixierbandes 26 in der Nut 28 enthält die Miniplastschiene 27 einstückig angeformte Fixierzapfen 30, die jeweils von einer Seite in die Öffnung der Nut 28 hineinragen und den Öffnungsquerschnitt verändern.

Wie aus **Fig. 11** ersichtlich, sind die verschiedenen Fixierzapfen 30 jeweils abwechselnd an der oberen Seitenkante 31 bzw. der unteren Seitenkante 32 der Nut 28 angeordnet, so dass das Fixierband 26 entsprechend leichter in die Nut 28 eingeführt werden kann.

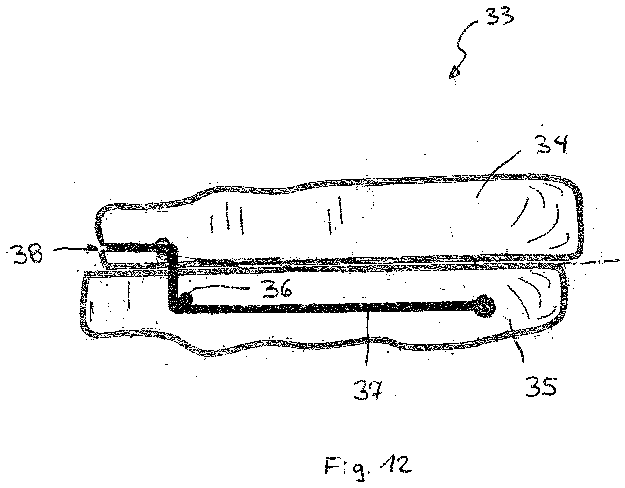

**Fig. 12** zeigt eine zweite Ausführungsform 33 einer Okklusionsschienenanordnung in seitlicher Ansicht. Der grundsätzliche Aufbau der Okklusionsschienenanordnung 33 mit zwei Miniplastschienen 34 und 35 entspricht dem Aufbau der Okklusionsschienenanordnung 01, wie sie in Fig. 2 dargestellt ist. Anders als bei der Okklusionsschienenanordnung 01 ist aber bei der Okklusionsschienenanordnung 33 an der mandibularen Miniplastschiene 35 ein Umlenkelement 36 vorgesehen, an dem ein Fixierband 37 nach dem Einhängen in die Nut 38 der maxillaren Miniplastschiene 34 umgelenkt werden kann. In Fig. 12 ist dabei nur das linke Umlenkelement 36 dargestellt. Auf der gegenüberliegenden Seite der mandibularen Miniplastschiene 35 befindet sich ein entsprechendes rechtes Umlenkelement. Durch die beiden an der mandibularen Miniplastschiene 35 vorgesehenen Umlenkelemente 36 kann die mandibulare Miniplastschiene 35, und damit der Unterkiefer, gegen die maxillare Miniplastschiene 34, und damit gegen den Oberkiefer, gezogen und insofern der Öffnungswinkel des Unterkiefers relativ zum Oberkiefer begrenzt werden.

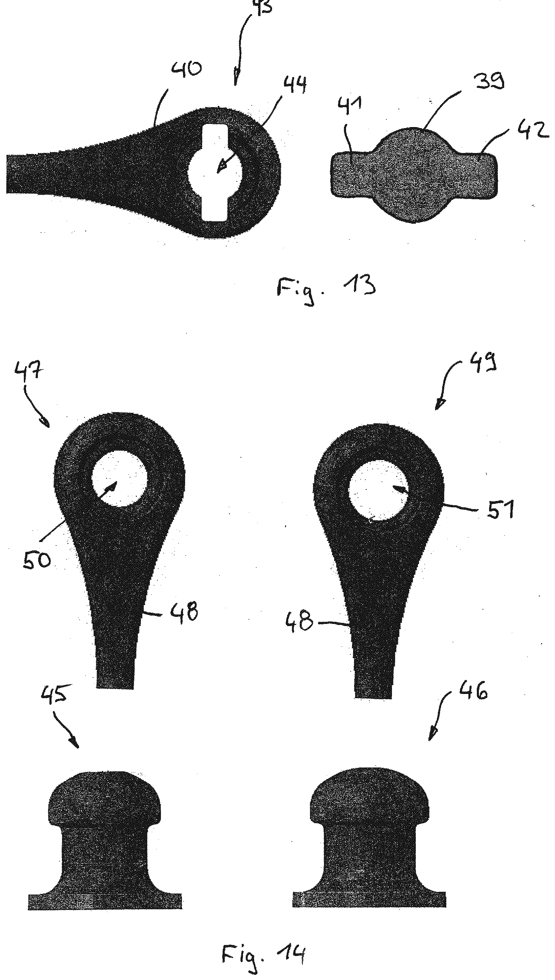

**Fig. 13** zeigt eine zweite Ausführungsform 39 eines Befestigungselements an einer mandibularen Miniplastschiene. Neben dem Befestigungselement 39 ist das Ende des zugehörigen Fixierbandes 40 dargestellt. Das Befestigungselement 39 ist in der Art eines Fixierzapfens ausgebildet und in Fig. 13 in Ansicht von oben gezeigt. An dem Befestigungselement 39 sind zwei Überstände 41 und 42 angeformt, die seitlich über den Hauptkörper des Fixierzapfens überstehen. Am Ende 43 des Fixierbandes 40 ist eine Öffnung 44 vorgesehen, deren Kontur der Kontur des Befestigungselements 39 im Bereich der Überstände 41 und 42 entspricht. Durch Verdrehen des Endes 43 um 90° kann der Fixierzapfen des Befestigungselements 39 in die Öffnung 44 eingeführt und dann durch Rückdrehen des Endes 43 um 90° fixiert werden. In ihrer Fixierstellung hintergreifen die Überstände 41 und 42 den Rand der Öffnung 44 und verhindern damit ein unerwünschtes Lösen des Fixierbandes 40 vom Befestigungselement 39.

**Fig. 14** zeigt eine dritte Ausführungsform von Befestigungselementen 45 bzw. 46, die jeweils in der Art von Befestigungszapfen ausgebildet sind. Dem linken Befestigungselement 45 ist dabei das linke Ende 47 eines Fixierbandes 48 zugeordnet. Dem rechten Befestigungselement 46 ist das rechte Ende 49 des Fixierbandes 48 zugeordnet. Die Öffnung 50 am linken Ende 47 des Fixierbandes 48 ist im Querschnitt kleiner als die Öffnung 51 am rechten Ende 49 des Fixierbandes 48. Entsprechend haben auch die Fixierzapfen am linken Befestigungselement 45 und am rechten Befestigungselement 46 jeweils einen unterschiedlichen Durchmesser, so dass die Öffnung 50 auf das Befestigungselement 45 und die Öffnung 51 auf das Befestigungselement 46 aufgeschoben und damit das Fixierband 48 rechts und links eingehängt werden kann. Aufgrund des unterschiedlichen Durchmessers der Öffnungen 50 und 51 ist dagegen das versehentliche Einhängen des linken Endes 47 am rechten Befestigungselement 46 ausgeschlossen, da der Durchmesserunterschied zu groß ist, als dass das linke Ende 47 mit seiner Öffnung 50 am rechten Befestigungselement 46 eingehängt werden könnte. Insofern ist ein lagefalsches Einhängen des Fixierbandes 48 an den beiden Befestigungselementen 45 und 46 ausgeschlossen.

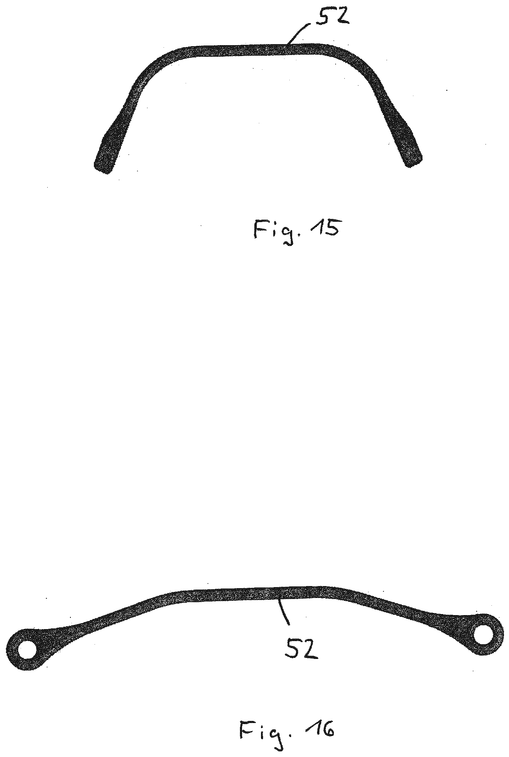

**Fig. 15** und **Fig. 16** zeigen eine Ausführungsform 52 eines vorgeformten Fixierbandes in Ansicht von oben bzw. in Ansicht von vorne. Aufgrund der bogenförmigen Vorformung des Fixierbandes 52 entspricht dessen Gestalt im belastungsfreien Zustand gerade der bogenförmigen Gestalt, die das Fixierband 52 bei Fixierung an einer Okklusionsschienenanordnung 01 einnimmt. Damit werden Spannungsbelastungen durch Verformung des Fixierbandes 52, wie sie bei nicht vorgeformten Fixierbändern auftreten, vermieden.

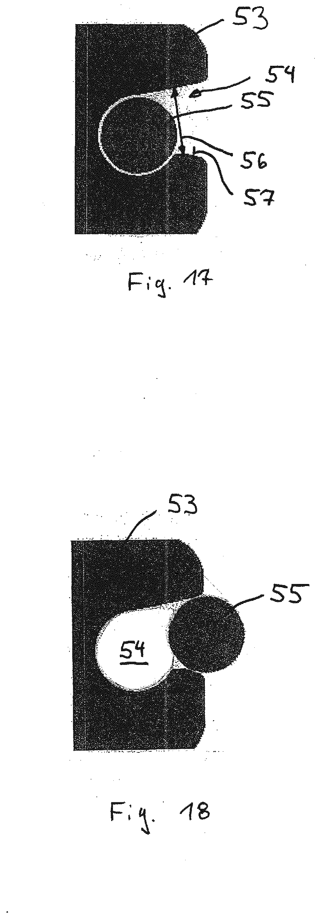

**Fig. 17** und **Fig. 18** zeigen einen Teilquerschnitt einer maxillaren Miniplastschiene 53 im Bereich der Nut 54 zur fixierenden Aufnahme eines im Querschnitt kreisförmigen Fixierbandes 55. Das Fixierband 55 ist aus einem quer zu seiner Längsachse elastisch verformbaren Kunststoffmaterial hergestellt. Die Öffnung 56 der Nut 54 ist in der Art einer Verengung ausgebildet, so dass der Öffnungsquerschnitt der Öffnung 56 kleiner als der Außendurchmesser des Fixierbandes 55 ist. Wird das Fixierband 55, wie in Fig. 18 schematisiert dargestellt, von außen in Richtung der Nut 54 gedrückt, so muss ein Verformungswiderstand überwunden werden, durch den das Fixierband 55 geringfügig abgeplattet wird, so dass es in die Nut 54 hineingleitet. Um dieses Einführen des Fixierbandes 55 in die Nut 54 zu erleichtern, ist an der nach außen weisenden Seite der als Verengung ausgebildeten Öffnung 56 eine Einführschräge 57 vorgesehen. Sobald das Fixierband 55 vollständig in der Nut 54 aufgenommen ist, bildet sich ein formschlüssiger Presssitz, so dass das Fixierband 55 nur noch unter Überwindung eines entsprechenden Reibungswiderstands relativ zur Nut 54 bewegt werden kann.

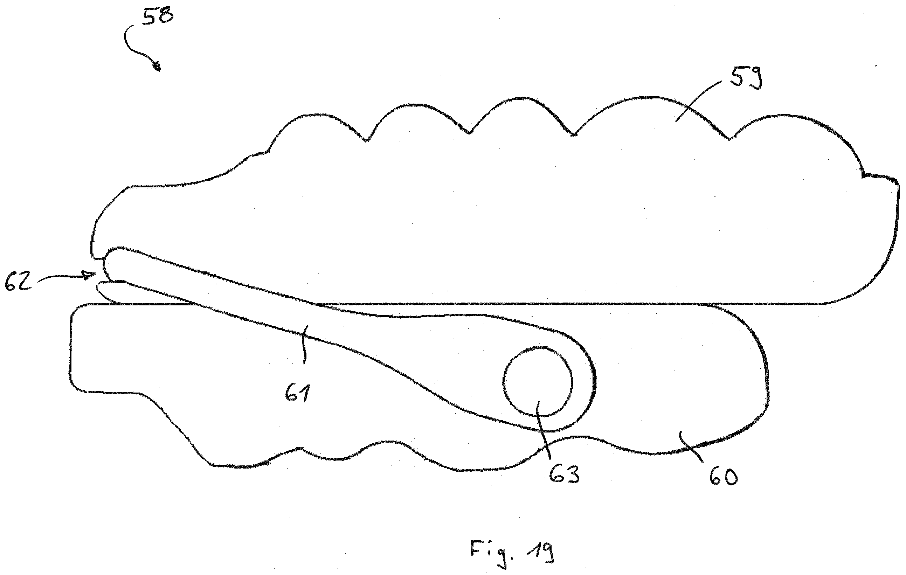

**Fig. 19** zeigt eine dritte Ausführungsform 58 einer Okklusionsschienenanordnung in seitlicher Ansicht. Der grundsätzliche Aufbau der Okklusionsschienenanordnung 58 mit zwei Miniplastschienen 59 und 60 entspricht dem Aufbau der Okklusionsschienenanordnung 01, wie sie in Fig. 2 dargestellt ist.

Bei der Okklusionsschienenanordnung 58 ist zur Verbindung der maxillaren Miniplastschiene 59 und der mandibularen Miniplastschiene 60 ein Fixierband 61 vorgesehen, das in einer Nut 62 der maxillaren Miniplastschiene 59 fixiert werden kann. Die Enden des Fixierbandes 61 werden an Befestigungselementen 63 und 64 befestigt. Die Befestigungselemente 63 und 64 sind einstückig aus Kunststoff an die mandibularen Miniplastschiene 60 angeformt und können im 3D-Druck aus Kunststoff hergestellt werden.

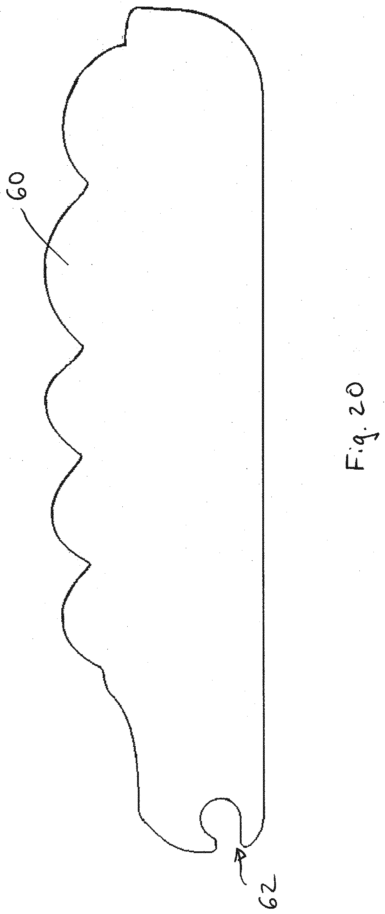

**Fig. 20** zeigt die maxillare Miniplastschiene 59 der Okklusionsschienenanordnung 58 mit der Nut 62 in seitlicher Ansicht.

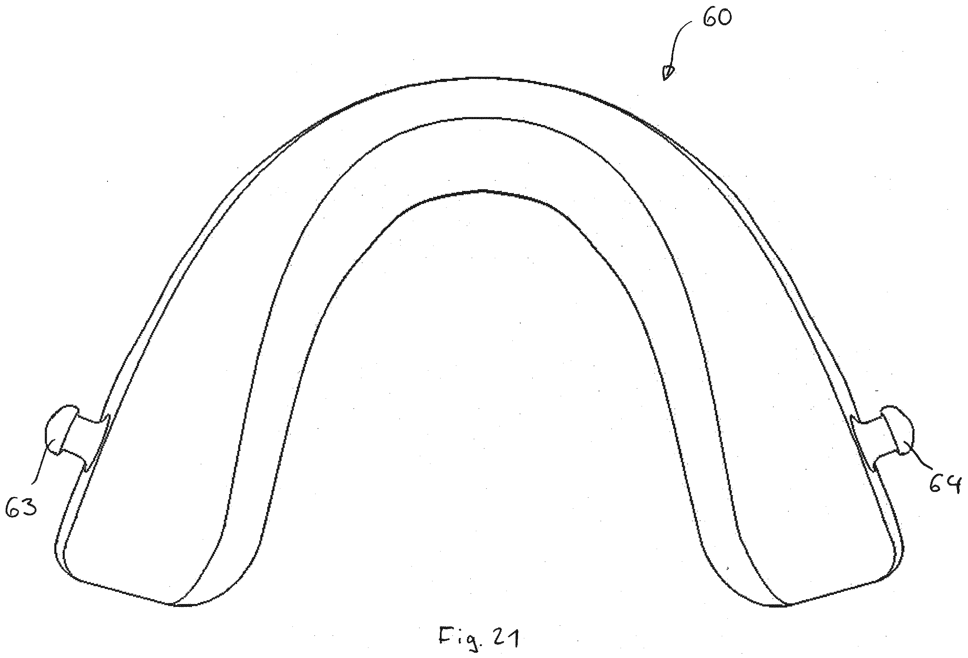

**Fig. 21** zeigt die mandibulare Miniplastschiene 60 mit den Befestigungselementen 63 und 64 in Ansicht von oben.

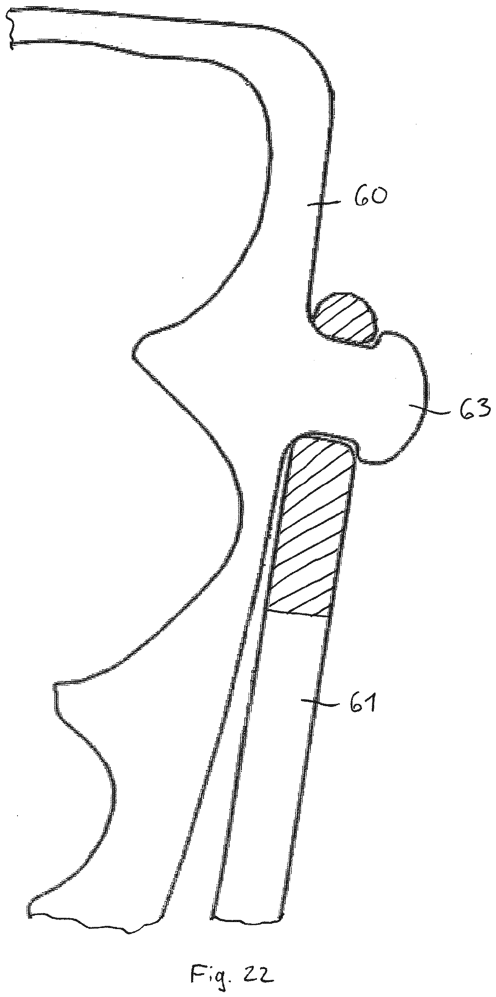

**Fig. 22** zeigt das Befestigungselement 63 der mandibularen Miniplastschiene mit daran befestigtem Fixierband 61 im Teilquerschnitt.

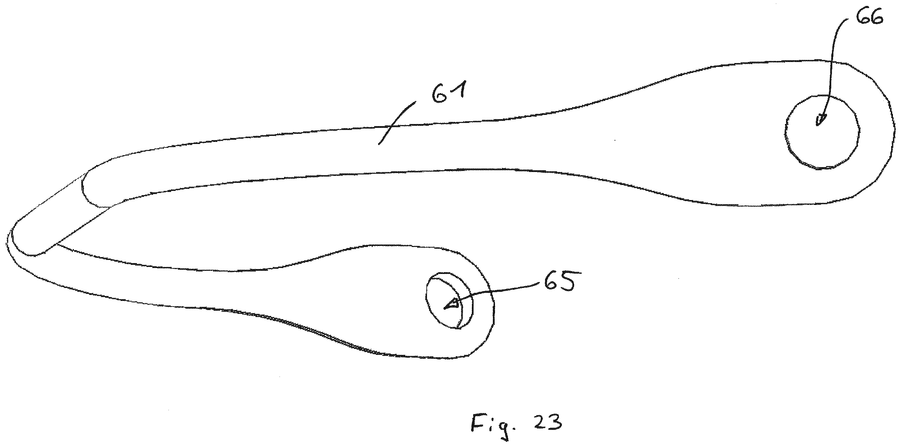

**Fig. 23** zeigt das vorgeformte Fixierband 61 der Okklusionsschienenanordnung 58 mit den Öffnungen 65 und 66 zum Einhängen an den Befestigungselementen 63 und 64 in seitlicher perspektivischer Ansicht.

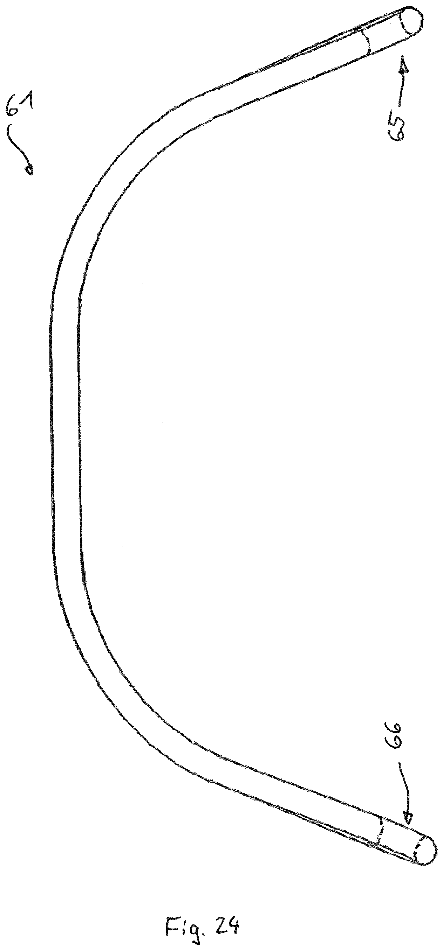

**Fig. 24** zeigt das vorgeformte Fixierband 58 mit den Öffnungen 65 und 66 zum Einhängen an den Befestigungselementen 63 und 64 in Ansicht von oben.

**Bezugszeichenliste**

| | | | |
|---|---|---|---|
| 01 | Okklusionsschienenanordnung | 39 | Befestigungselement |
| 02 | Maxillare Miniplastschiene | 40 | Fixierband |
| 03 | Mandibulare Miniplastschiene | 41 | Überstand |
| 04 | Transversalebene | 42 | Überstand |
| 05 | Fixierband | 43 | Ende (Fixierband) |
| 06 | Ende (Fixierband) | 44 | Öffnung (Fixierband) |
| 07 | Ausnehmung (Fixierband) | 45 | Linkes Befestigungselement |
| 08 | Befestigungselement | 46 | Rechtes Befestigungselement |
| 09 | Backenzahnbereich | 47 | Linkes Ende (Fixierband) |
| 10 | Schneidezahnbereich | 48 | Fixierband |
| 11 | Führungseinrichtung | 49 | Rechtes Ende (Fixierband) |
| 12 | Mittelabschnitt (Fixierband) | 50 | Öffnung (Fixierband) |
| 13 | Nut | 51 | Öffnung (Fixierband) |
| 14 | Ende (Nut) | 52 | Vorgeformtes Fixierband |
| 15 | Freier Längenabschnitt (Fixierband) | 53 | Maxillare Miniplastschiene |
| 16 | Fixierzapfen | 54 | Nut |
| 17 | Sicherungselement | 55 | Fixierband |
| 18 | Mandibulare Miniplastschiene | 56 | Öffnung mit Verengung |
| 19 | Abdeckelement | 57 | Einführschräge |
| 20 | Glattflächige Seitenfläche (Abdeckelement) | 58 | Okklusionsschienenanordnung |
| 21 | Öffnung (Nut) | 59 | Maxillare Miniplastschiene |
| 22 | Fixiersteg | 60 | Mandibulare Miniplastschiene |
| 23 | Fixiersteg | 61 | Fixierband |
| 24 | Maxillare Miniplastschiene | 62 | Nut |
| 25 | Nut | 63 | Befestigungselement |
| 26 | Fixierband | 64 | Befestigungselement |
| 27 | Maxillare Miniplastschiene | 65 | Öffnung |
| 28 | Nut | 66 | Öffnung |
| 29 | Fixiereinrichtung | | |
| 30 | Fixierzapfen | | |
| 31 | Obere Seitenkante (Nut) | | |
| 32 | Untere Seitenkante (Nut) | | |
| 33 | Okklusionsschienenanordnung | | |
| 34 | Maxillare Miniplastschiene | | |
| 35 | Mandibulare Miniplastschiene | | |
| 36 | Umlenkelement | | |
| 37 | Fixierband | | |
| 38 | Nut | | |

## Patentansprüche

1. Okklusionsschienenanordnung (01, 33, 58), insbesondere zur Schlafapnoe-Therapie, mit einer auf der maxillaren Zahnreihe anordenbaren maxillaren Miniplastschiene (02, 24, 27, 34, 53, 59) und einer auf der mandibularen Zahnreihe anordenbaren mandibularen Miniplastschiene (03, 18, 35, 60), wobei die mandibulare Miniplastschiene (03, 18, 35, 60) in einer Transversalebene (04) gegen die maxillaren Miniplastschiene (02, 24, 27, 34, 53, 59) zur Anlage gebracht werden kann, und wobei die mandibulare Miniplastschiene (03, 18, 35, 60) in der Transversalebene (04) labial in X-Richtung und bukkal in Y-Richtung relativ zur maxillaren Miniplastschiene (02, 24, 27, 34, 53, 59) verschiebbar ist, und wobei die Okklusionsschienenanordnung (01, 33, 58) ein Positionierungsmittel umfasst, mit dem die Stellung der mandibularen Miniplastschiene (03, 18, 35, 60) relativ zur maxillaren Miniplastschiene (02, 24, 27, 34, 53, 59) in X-Richtung definierbar ist,
wobei das Positionierungsmittel in der Art eines Zugkräfte übertragenden Fixierbandes (05, 26, 37, 40, 48, 52, 55, 61) ausgebildet ist, wobei die beiden Enden (06, 43, 47, 49) des Fixierbandes (05, 26, 37, 40, 48, 52, 55, 61) an zwei Befestigungselementen (08, 39, 45, 46, 63, 64) fixierbar sind, und wobei die Befestigungselemente (08, 39, 45, 46, 63, 64) im linken und rechten Backenzahnbereich (09) der mandibularen Miniplastschiene (03, 18, 35, 60) angeordnet sind, und wobei ein Mittelabschnitt (12) des Fixierbandes (05, 26, 37, 40, 48, 52, 55, 61) an einer Führungseinrichtung (11, 29) geführt ist, und wobei die Führungseinrichtung (11, 29) im Schneidezahnbereich (10) der maxillaren Miniplastschiene (02, 24, 27, 34, 53, 59) angeordnet ist,
**dadurch gekennzeichnet, dass**
die Führungseinrichtung (11, 29) an der maxillaren Miniplastschiene (02, 24, 27, 34, 53, 59) als eine Nut (13, 25, 28, 38, 54, 62) ausgebildet ist, die im Schneidezahnbereich (10) der maxillaren Miniplastschiene (02, 24, 27, 34, 53, 59) labial angeordnet ist, wobei die Nut (13, 25, 28, 38, 54, 62) das Fixierband (05, 26, 37, 40, 48, 52, 55, 61) zumindest teilweise aufnimmt.

2. Okklusionsschienenanordnung nach Anspruch 1,
**dadurch gekennzeichnet,**
**dass** die beiden Befestigungselemente (08) einstückig an die mandibulare Miniplastschiene (03) angeformt oder eingeformt sind und/oder dass die Führungseinrichtung (11, 29) einstückig an die maxillare Miniplastschiene (02, 24, 27) angeformt oder eingeformt ist.

3. Okklusionsschienenanordnung nach Anspruch 2,
**dadurch gekennzeichnet,**
**dass** die beiden Befestigungselemente (08) aus dem gleichen Kunststoffmaterial wie die mandibulare Miniplastschiene (03) gebildet sind und/oder dass die Führungseinrichtung (11, 29) aus dem gleichen Kunststoffmaterial wie die maxillare Miniplastschiene (02, 24, 27) gebildet ist.

4. Okklusionsschienenanordnung nach Anspruch 3,
**dadurch gekennzeichnet,**
**dass** die mandibulare Miniplastschiene (03) zusammen mit den beiden einstückig angeformten oder eingeformten Befestigungselementen (08) in einem 3D-Kunststoffdruckverfahren hergestellt ist und/oder dass die maxillare Miniplastschiene (02, 24, 27) zusammen mit der einstückig angeformten oder eingeformten Führungseinrichtung (11, 29) in einem 3D-Kunststoffdruckverfahren hergestellt ist oder dass die mandibulare Miniplastschiene (03) zusammen mit den beiden einstückig angeformten oder eingeformten Befestigungselementen (08) in einem Mehrachsfräsverfahren hergestellt ist und/oder dass die maxillare Miniplastschiene (02, 24, 27) zusammen mit der einstückig angeformten oder eingeformten Führungseinrichtung (11, 29) in einem Mehrachsfräsverfahren hergestellt ist.

5. Okklusionsschienenanordnung nach einem der Ansprüche 1 bis 4,
**dadurch gekennzeichnet,**
**dass** das Fixierband (05, 26, 37, 40, 48, 52, 55, 61) längenverstellbar ist, dass das Fixierband (05, 26, 55) aus Kunststoff hergestellt ist, dass das Fixierband (05, 26) in Richtung seiner Längsachse hochsteif ausgebildet ist und/oder dass das Fixierband (55) quer zu seiner Längsachse elastisch verformbar ausgebildet ist.

6. Okklusionsschienenanordnung nach einem der Ansprüche 1 bis 5,
**dadurch gekennzeichnet,**
**dass** zwischen den beiden seitlichen Enden (14) der Nut (13, 25, 28) in der maxillaren Miniplastschiene (02, 24, 27) und den beiden Befestigungselementen (08) an der mandibularen Miniplastschiene (03) jeweils ein Abstand vorhanden ist, in dem das Fixierband (05, 26) mit einem freien Längenabschnitt (15) ungeführt verläuft, wobei die beiden freien Längenabschnitte (15) des Fixierbandes eine Verschiebung der mandibulare Miniplastschiene (03) relativ zur maxillaren Miniplastschiene (02, 24, 27) zumindest in Y-Richtung ermöglichen und/oder dass zwischen den beiden seitlichen Enden der Nut (38) in der maxillaren Miniplastschiene (34) und den beiden Befestigungselementen an der mandibularen Miniplastschiene (35) jeweils ein Abstand vorhanden ist, wobei an der mandibularen Miniplastschiene (35) ein rechtes und ein linkes Umlenkelement (36) vorgesehen sind, an denen das Fixierband (37) umgelenkt wird und dadurch die vertikale Relativbewegung zwischen der maxillaren Miniplastschiene (34) und der mandibularen Miniplastschiene (35) begrenzt wird.

7. Okklusionsschienenanordnung nach einem der Ansprüche 1 bis 6,
**dadurch gekennzeichnet,**
**dass** an der Öffnung (56) der Nut (54) eine oder mehrere Verengungen vorgesehen sind, durch die das, insbesondere im Querschnitt kreisförmige, Fixierband (55) formschlüssig in der Nut (54) fixiert wird.

8. Okklusionsschienenanordnung Anspruch 7,
**dadurch gekennzeichnet,**
**dass** an der nach außen weisenden Seite der Öffnung (56) mit Verengung eine Einführschräge (57) vorgesehen ist, durch die das Fixierband (55) in die Nut (54) geleitet wird.

9. Okklusionsschienenanordnung nach einem der Ansprüche 1 bis 8,
**dadurch gekennzeichnet,**
**dass** an der Öffnung der Nut (25, 28) Fixerzapfen (30) und/oder Fixierstege (22, 23) vorgesehen sind, die die Öffnung der Nut (25, 28) verengen.

10. Okklusionsschienenanordnung nach Anspruch 9,
**dadurch gekennzeichnet,**
**dass** an der Öffnung der Nut (28) zumindest drei Fixerzapfen (30) vorgesehen sind, wobei jeweils benachbarte Fixerzapfen (30) abwechselnd von den beiden Seitenkanten (31, 32) der Nut (28) in die Öffnung ragen und/oder dass an der Öffnung der Nut (25) zumindest ein Fixiersteg (22, 23) vorgesehen ist, wobei sich der Fixiersteg (22, 23) durchgehend zwischen den beiden seitlichen Enden (14) der Nut (25) erstreckt.

11. Okklusionsschienenanordnung nach einem der Ansprüche 1 bis 10,
**dadurch gekennzeichnet,**
**dass** der Querschnitt der Nut (54) zumindest geringfügig kleiner als der Querschnitt des Fixierbandes (55) ist, wobei das Fixierband (55) durch den dadurch gebildeten Presssitz in der Nut (54) fixiert wird, dass das Fixierband (55) einen kreisförmigen Querschnitt aufweist, dass der Querschnitt der Nut (13, 25, 28) zumindest geringfügig größer als der Querschnitt des Fixierbandes (05, 26) ist und das Fixierband (05, 26) entlang seiner Längsachse widerstandsfrei in der Nut (13, 25, 28) verschoben werden kann, dass das Fixierband (26) einen rechteckförmigen Querschnitt aufweist, wobei das Fixierband (26) im Schneidezahnbereich mit der Längsseite des rechteckförmigen Querschnitts parallel zum Zahnbogen verläuft und/oder dass die Breite des Fixierbandes (26) größer ist als der Öffnungsquerschnitt der Nut (25), wobei die Höhe des Fixierbandes (26) kleiner ist als der Öffnungsquerschnitt der Nut (25).

12. Okklusionsschienenanordnung nach einem der Ansprüche 1 bis 11,
**dadurch gekennzeichnet,**
**dass** die Befestigungselemente (08) an der mandibularen Miniplastschiene (03) in der Art von Fixierzapfen (16) ausgebildet sind, die im rechten und linken Backenzahnbereich (09) der mandibularen Miniplastschiene (03) seitlich überstehen, wobei die Enden (06) des Fixierbandes (05) an den Fixierzapfen (16) fixierend eingehängt werden können.

13. Okklusionsschienenanordnung nach Anspruch 12,
**dadurch gekennzeichnet,**
**dass** am freien Ende der Fixierzapfen (16) ein Sicherungselement (17), insbesondere eine Verdickung, vorgesehen ist, mit dem der Sitz des Fixierbandes (05, 26) am Fixierzapfen (16) gesichert wird.

14. Okklusionsschienenanordnung nach einem der Ansprüche 1 bis 13,
**dadurch gekennzeichnet,**
**dass** am freien Ende der Fixierzapfen (39) jeweils zumindest ein Überstand (41, 42) vorgesehen ist, wobei in einer Montageposition des Fixierbandes (40) der Überstand (41, 42) in eine Öffnung (44) am Ende (43) des Fixierbandes (40) eingeführt werden kann, und wobei in einer Fixierposition des Fixierbandes (40) der Überstand (41, 42) die Öffnung (44) am Ende (43) des Fixierbandes (40) fixierend hintergreift, dass ein Sicherungselement am rechten Fixierzapfen (46) eine andere Form als ein Sicherungselement am linken Fixierzapfen (45) aufweist, insbesondere dass die Verdickung am rechten Fixierzapfen (46) einen anderen Durchmesser aufweist als die Verdickung am linken Fixierzapfen (45), wobei die Öffnung am linken Ende (47) des Fixierbandes (48) auf die Form des Sicherungselements am linken Fixierzapfen (45) und die Öffnung am rechten Ende (49) des Fixierbandes (48) auf die Form des Sicherungselements am rechten Fixierzapfen (46) abgestimmt ist, dass benachbart zu den Befestigungselementen (08) an der mandibularen Miniplastschiene (03) jeweils glattflächige Abdeckelemente (20) vorgesehen sind, die im Backenzahnbereich (09) der mandibularen Miniplastschiene (03) überstehen und die Befestigungselemente (08) seitlich zumindest teilweise abdecken und/oder dass auf den beiden Miniplastschienen (34, 35) Markierungen angebracht sind, die die Position der beiden Miniplastschienen (34, 35) relativ zueinander kennzeichnen.

## Claims

1. An occlusal splint arrangement (01, 33,58), in particular for sleep apnoea therapy, having a maxillary miniplast splint (02, 24, 27, 34, 53, 59) to be disposed on the maxillary row of teeth and a mandibular miniplast splint (03, 18, 35, 60) to be disposed on the mandibular row of teeth, the mandibular miniplast splint (03, 18, 35, 60) being brought into contact with the maxillary miniplast splint (02, 24, 27, 34, 53, 59) in a transverse plane (04), and the mandibular miniplast splint (03, 18, 35, 60) being displaced labially in the x-direction and buccally in the y-direction in the transverse plane (04) with respect to the maxillary miniplast splint (02, 24, 27, 34, 53, 59), and the occlusal splint arrangement (01, 33, 58) comprising a positioning means by means of which the position of the mandibular miniplast splint (03, 18, 35, 60) with respect to the maxillary miniplast splint (02, 24, 27, 34, 53, 59) is defined in the x-direction,
wherein the positioning means is designed like a fixing band (05, 26, 37, 40, 48, 52, 55, 61) transmitting tensile forces, the two ends (06, 43, 47, 49) of the fixing band (05, 26, 37, 40, 48, 52, 55, 61) being fixed to two fastening elements (08, 39, 45, 46, 63, 64), and the fastening elements (08, 39, 45, 46, 63, 64) being disposed in the left and the right molar region (09) of the mandibular miniplast splint (03, 18, 35, 60), and wherein a middle section (12) of the fixing band (05, 26, 37, 40, 48, 52, 55) being guided at a guide means (11, 29), and the guide means (11, 29) being disposed in the incisor region (10) of the maxillary miniplast splint (02, 24, 27, 34, 53, 59),
**characterized in that**
the guide means (11, 29) at the maxillary miniplast splint (02, 24, 27, 34, 53, 59) is designed like a groove (13, 25, 28, 38, 54, 62) which is disposed labially in the incisor region (10) of the maxillary miniplast splint (02, 24, 27, 34, 53, 59), the groove (13, 25, 28, 38, 54, 62) at least partially accommodating the fixing band (05, 26, 37, 40, 48, 52, 55, 61),

2. The occlusal splint arrangement according to claim 1,
**characterised in that**
the two fastening elements (08) are integrally moulded on or into the mandibular miniplast splint (03) and/or **in that** the guide means (11, 29) is integrally moulded on or into the maxillary miniplast splint (02, 24, 27).

3. The occlusal splint arrangement according to claim 2,
**characterised in that**
the two fastening elements (08) are made of the same plastic material as the mandibular miniplast splint (03) and/or **in that** the guide means (11, 29) is made of the same plastic material as the maxillary miniplast splint (02, 24, 27).

4. The occlusal splint arrangement according to claim 3,
**characterised in that**
the mandibular miniplast splint (03), together with the two fastening elements (08) integrally moulded thereon or thereinto, is produced in a 3D plastic printing process and/or **in that** the maxillary miniplast splint (02, 24, 27), together with the guide means (11, 29) integrally moulded thereon or thereinto, is produced in a 3D plastic printing process or **in that** the mandibular miniplast splint (03), together with the two fastening elements (08) integrally moulded thereon or thereinto, is produced in a multi-axis milling process and/or **in that** the maxillary miniplast splint (02, 24, 27), together with the guide means (11, 29) integrally moulded thereon or thereinto, is produced in a multi-axis milling process.

5. The occlusal splint arrangement according to any one of claims 1 to 4,
**characterised in that**
the fixing band (05, 26, 37, 40, 48, 52, 55, 61) is adjustable in length, **in that** the fixing band (05, 26, 55) is made of a plastic material, **in that** the fixing band (05, 26) is designed highly rigid in the direction of its longitudinal axis, and/or **in that** the fixing band (55) is designed elastically deformable transversely to its longitudinal axis.

6. The occlusal splint arrangement according to any one of claim 1 to 5,
**characterised in that**
between each one of the two lateral ends (14) of the groove (13, 25, 28) in the maxillary miniplast splint (02, 24, 27) and the respective one of the two fastening elements (08) at the mandibular miniplast splint (03) a distance is present, in which the fixing band (05, 26) runs unguided with a free length section (15), the two free length sections (15) of the fixing band allowing a displacement of the mandibular miniplast splint (03) with respect to the maxillary miniplast splint (02, 24, 27) at least in the y-direction, and/or **in that** between each one of the two lateral ends of the groove (38) in the maxillary miniplast splint (34) and the respective one of the two fastening elements at the mandibular miniplast splint (35) a distance is present, a right and a left deflection element (36) being provided at the mandibular miniplast splint (35), at which (36) the fixing band (37) is deflected and by which the vertical relative movement between the maxillary miniplast splint (34) and the mandibular miniplast splint (35) is limited.

7. The occlusal splint arrangement according to any one of claims 1 to 6,
**characterised in that**
one or more constrictions are provided at the opening (56) of the groove (54), by means of which the fixing band (55), which is in particular circular in the cross section, is fixed in a positive locking manner in the groove (54).

8. The occlusal splint arrangement according to claim 7,
**characterised in that**
at the outward facing side of the opening (56) having the constriction an insertion chamfer (57) is provided, by means of which (57) the fixing band (55) is guided into the groove (54).

9. The occlusal splint arrangement according to any one of claims 1 to 8,
**characterised in that**
at the opening of the groove (25, 28) fixing pins (30) and/or fixing webs (22, 23) which constrict the opening of the groove (25, 28) are provided.

10. The occlusal splint arrangement according to claim 9,
**characterised in that**
at least three fixing pins (30) are provided at the opening of the groove (28), fixing pins (30) which are respectively adjacent to each other projecting alternately from the two side edges (31, 32) of the groove (28) into the opening, and/or **in that** at least one fixing web (22, 23) is provided at the opening of the groove (25), the fixing web (22, 23) extending continuously between the two lateral ends (14) of the groove (25).

11. The occlusal splint arrangement according to any one of claims 1 to 10,
**characterised in that**
the cross section of the groove (54) is at least slightly smaller than the cross section of the fixing band (55), the fixing band (55) being fixed in the groove (54) by the press fit formed thereby, **in that** the fixing band (55) has a circular cross section, **in that** the cross section of the groove (13, 25, 28) is at least slightly larger than the cross section of the fixing band (05, 26) and the fixing band (05, 26) is displaced along its longitudinal axis without resistance in the groove (13, 25, 28), **in that** the fixing band (26) has a rectangular cross section, the fixing band (26) running in the incisor region with the longitudinal side of the rectangular cross section parallel to the dental arch, and/or **in that** the width of the fixing band (26) is larger than the opening cross section of the groove (25), the height of the fixing band (26) being smaller than the opening cross section of the groove (25).

12. The occlusal splint arrangement according to any one of claims 1 to 11,
**characterised in that**
the fastening elements (08) at the mandibular miniplast splint (03) are designed like fixing pins (16) which project laterally in the right and the left molar region (09) of the mandibular miniplast splint (03), the ends (06) of the fixing band (05) being hooked onto the fixing pins (16) in a fixing manner.

13. The occlusal splint arrangement according to claim 12,
**characterised in that**
a securing element (17), in particular an enlargement, by means of which the fit of the fixing band (05, 26) at the fixing pin (16) is secured, is provided at the free end of the fixing pins (16).

14. The occlusal splint arrangement according to any one of claims 1 to 13,
**characterised in that**
at each free end of the fixing pins (39) at least one projection (41, 42) is provided, the projection (41, 42), in a mounting position of the fixing band (40), being inserted into an opening (44) at the end (43) of the fixing band (40), and the projection (41, 42), in a fixing position of the fixing band (40), engaging in a fixing manner behind the opening (44) at the end (43) of the fixing band (40), **in that** a securing element at the right fixing pin (46) has a different shape than a securing element at the left fixing pin (45), in particular **in that** the enlargement at the right fixing pin (46) has a different diameter than the enlargement at the left fixing pin (45), the opening at the left end (47) of the fixing band (48) being adapted to the shape of the securing element at the left fixing pin (45) and the opening at the right end (49) of the fixing band (48) being adapted to the shape of the securing element at the right fixing pin (46), **in that** smooth cover elements (20) are provided adjacent to each fastening element (08) at the mandibular miniplast splint (03), said cover elements (20) projecting in the molar region (09) of the mandibular miniplast splint (03) and laterally covering the fastening elements (08) at least partially, and/or **in that** markings that indicate the position of the two miniplast splints (34, 35) with respect to each other are attached to the two miniplast splints (34, 35).

## Revendications

1. Dispositif de gouttière occlusale (01, 33, 58), en particulier destiné au traitement de l'apnée du sommeil, comprenant une gouttière maxillaire miniplast (02, 24, 27, 34, 53, 59) apte à être disposée sur l'arcade dentaire maxillaire et une gouttière mandibulaire miniplast (03, 18, 35, 60) apte à être disposée sur l'arcade dentaire mandibulaire, la gouttière mandibulaire miniplast (03, 18, 35, 60) pouvant venir en appui, dans un plan transversal (04), contre la gouttière maxillaire miniplast (02, 24, 27, 34, 53, 59), la gouttière mandibulaire miniplast (03, 18, 35, 60) étant déplaçable dans le plan transversal (04) selon une direction labiale X et selon une direction buccale Y par rapport à la gouttière maxillaire miniplast (02, 24, 27, 34, 53, 59), et le dispositif de gouttière occlusale (01, 33, 58) comprenant un moyen de positionnement au moyen duquel la position de la gouttière mandibulaire miniplast (03, 18, 35, 60) par rapport à la gouttière maxillaire miniplast (02, 24, 27, 34, 53, 59) est définissable en direction X,
ledit moyen de positionnement étant réalisé sous la forme d'une bande de fixation (05, 26, 37, 40, 48, 52, 55, 61) transmettant des efforts de traction, les deux extrémités (06, 43, 47, 49) de la bande de fixation (05, 26, 37, 40, 48, 52, 55, 61) pouvant être fixées à deux éléments de fixation (08, 39, 45, 46, 63, 64), lesdits éléments de fixation (08, 39, 45, 46, 63, 64) étant disposés dans les régions molaires gauche et droite (09) de la gouttière mandibulaire miniplast (03, 18, 35, 60), une portion médiane (12) de la bande de fixation (05, 26, 37, 40, 48, 52, 55, 61) étant guidée par un dispositif de guidage (11, 29), ledit dispositif de guidage (11, 29) étant disposé dans la région des incisives (10) de la gouttière maxillaire miniplast (02, 24, 27, 34, 53, 59),
**caractérisé en ce que**
le dispositif de guidage (11, 29) sur la gouttière maxillaire miniplast (02, 24, 27, 34, 53, 59) est réalisé sous forme d'une rainure (13, 25, 28, 38, 54, 62) disposée de manière labiale dans la région des incisives (10) de la gouttière maxillaire miniplast (02, 24, 27, 34, 53, 59), la rainure (13, 25, 28, 38, 54, 62) recevant au moins partiellement la bande de fixation (05, 26, 37, 40, 48, 52, 55, 61).

2. Dispositif de gouttière occlusale selon la revendication 1,
**caractérisé en ce que**
les deux éléments de fixation (08) sont réalisés d'une seule pièce avec la gouttière mandibulaire miniplast (03), par moulage ou formation intégrée, et/ou **en ce que** le dispositif de guidage (11, 29) est réalisé d'une seule pièce avec la gouttière maxillaire miniplast (02, 24, 27), par moulage ou formation intégrée.

3. Dispositif de gouttière occlusale selon la revendication 2,
**caractérisé en ce que**
les deux éléments de fixation (08) sont constitués du même matériau plastique que la gouttière mandibulaire miniplast (03) et/ou **en ce que** le dispositif de guidage (11, 29) est constitué du même matériau plastique que la gouttière maxillaire miniplast (02, 24, 27).

4. Dispositif de gouttière occlusale selon la revendication 3,
**caractérisé en ce que**
la gouttière mandibulaire miniplast (03) est fabriquée, avec les deux éléments de fixation (08) réalisés d'une seule pièce, par un procédé d'impression 3D de matière plastique, et/ou **en ce que** la gouttière maxillaire miniplast (02, 24, 27) est fabriquée, avec le dispositif de guidage (11, 29) réalisé d'une seule pièce, par un procédé d'impression 3D de matière plastique, ou **en ce que** la gouttière mandibulaire miniplast (03) est fabriquée, avec les deux éléments de fixation (08) réalisés d'une seule pièce, par un procédé d'usinage par fraisage multiaxes, et/ou **en ce que** la gouttière maxillaire miniplast (02, 24, 27) est fabriquée, avec le dispositif de guidage (11, 29) réalisé d'une seule pièce, par un procédé d'usinage par fraisage multiaxes.

5. Dispositif de gouttière occlusale selon l'une quelconque des revendications 1 à 4,
**caractérisé en ce que**
la bande de fixation (05, 26, 37, 40, 48, 52, 55, 61) est réglable en longueur, **en ce que** la bande de fixation (05, 26, 55) est réalisée en matière plastique, **en ce que** la bande de fixation (05, 26) présente une rigidité élevée selon son axe longitudinal et/ou **en ce que** la bande de fixation (55) est élastiquement déformable transversalement à son axe longitudinal.

6. Dispositif de gouttière occlusale selon l'une quelconque des revendications 1 à 5,
**caractérisé en ce qu'**il
existe, entre chacune des deux extrémités latérales (14) de la rainure (13, 25, 28) dans la gouttière maxillaire miniplast (02, 24, 27) et chacun des deux éléments de fixation (08) sur la gouttière mandibulaire miniplast (03), un espace dans lequel la bande de fixation (05, 26) se prolonge sans guidage avec une portion de longueur libre (15), les deux portions de longueur libre (15) de la bande de fixation permettant un déplacement de la gouttière mandibulaire miniplast (03) par rapport à la gouttière maxillaire miniplast (02, 24, 27) au moins selon la direction Y, et/ou **en ce qu'**il existe, entre les deux extrémités latérales de la rainure (38) dans la gouttière maxillaire miniplast (34) et les deux éléments de fixation sur la gouttière mandibulaire miniplast (35), un espace respectif, un élément de renvoi droit et un élément de renvoi gauche (36) étant prévus sur la gouttière mandibulaire miniplast (35), sur lesquels la bande de fixation (37) est renvoyée, limitant ainsi le mouvement relatif vertical entre la gouttière maxillaire miniplast (34) et la gouttière mandibulaire miniplast (35).

7. Dispositif de gouttière occlusale selon l'une quelconque des revendications 1 à 6,
**caractérisé en ce qu'**un
ou plusieurs rétrécissements sont prévus au niveau de l'ouverture (56) de la rainure (54), au travers desquels la bande de fixation (55), en particulier de section sensiblement circulaire, est fixée par complémentarité de forme dans la rainure (54).

8. Dispositif de gouttière occlusale selon la revendication 7,
**caractérisé en ce qu'**une
rampe d'introduction (57) est prévue sur le côté externe de l'ouverture (56) présentant un rétrécissement, au moyen de laquelle la bande de fixation (55) est guidée dans la rainure (54).

9. Dispositif de gouttière occlusale selon l'une quelconque des revendications 1 à 8,
**caractérisé en ce qu'**à
l'ouverture de la rainure (25, 28) sont prévus des tenons de fixation (30) et/ou des nervures de fixation (22, 23) qui rétrécissent l'ouverture de la rainure (25, 28).

10. Dispositif de gouttière occlusale selon la revendication 9,
**caractérisé en ce qu'**au
moins trois tenons de fixation (30) sont prévus à l'ouverture de la rainure (28), des tenons de fixation (30) adjacents faisant saillie alternativement depuis les deux bords latéraux (31, 32) de la rainure (28) dans l'ouverture, et/ou **en ce qu'**au moins une nervure de fixation (22, 23) est prévue à l'ouverture de la rainure (25), ladite nervure de fixation (22, 23) s'étendant de manière continue entre les deux extrémités latérales (14) de la rainure (25).

11. Dispositif de gouttière occlusale selon l'une quelconque des revendications 1 à 10,
**caractérisé en ce que**
la section de la rainure (54) est au moins légèrement inférieure à la section de la bande de fixation (55), la bande de fixation (55) étant immobilisée dans la rainure (54) par l'ajustement serré ainsi obtenu, **en ce que** la bande de fixation (55) présente une section circulaire, **en ce que** la section de la rainure (13, 25, 28) est au moins légèrement supérieure à la section de la bande de fixation (05, 26) et que la bande de fixation (05, 26) peut se déplacer sans résistance le long de son axe longitudinal dans la rainure (13, 25, 28), **en ce que** la bande de fixation (26) présente une section rectangulaire, ladite bande (26) s'étendant, dans la région des incisives, avec le grand côté de la section rectangulaire parallèle à l'arcade dentaire et/ou **en ce que** la largeur de la bande (26) est supérieure à la section d'ouverture de la rainure (25), la hauteur de la bande (26) étant inférieure à la section d'ouverture de la rainure (25).

12. Dispositif de gouttière occlusale selon l'une quelconque des revendications 1 à 11,
**caractérisé en ce que**
les éléments de fixation (08) sur la gouttière mandibulaire miniplast (03) sont réalisés sous forme de tenons de fixation (16) faisant saillie latéralement dans les régions molaires droite et gauche (09) de la gouttière mandibulaire miniplast (03), les extrémités (06) de la bande de fixation (05) pouvant être accrochées de manière fixe aux tenons de fixation (16).

13. Dispositif de gouttière occlusale selon la revendication 12,
**caractérisé en ce qu'**à
l'extrémité libre des tenons de fixation (16) est prévu un élément de sécurité (17), en particulier un épaississement, au moyen duquel la tenue de la bande de fixation (05, 26) sur le tenon de fixation (16) est assurée.

14. Dispositif de gouttière occlusale selon l'une quelconque des revendications 1 à 13,
**caractérisé en ce qu'**au
moins une saillie (41, 42) est prévue à l'extrémité libre de chacun des tenons de fixation (39), la saillie (41, 42) pouvant, dans une position de montage de la bande de fixation (40), être introduite dans une ouverture (44) à l'extrémité (43) de la bande de fixation (40), et, dans une position de fixation de ladite bande (40), venir s'engager en retenue derrière l'ouverture (44) à l'extrémité (43) de la bande (40), **en ce qu'**un élément de sécurité sur le tenon de fixation droit (46) présente une forme différente de celui sur le tenon de fixation gauche (45), en particulier **en ce que** l'épaississement sur le tenon droit (46) présente un diamètre différent de celui sur le tenon gauche (45), l'ouverture à l'extrémité gauche (47) de la bande de fixation (48) étant adaptée à la forme de l'élément de sécurité du tenon gauche (45) et l'ouverture à l'extrémité droite (49) de la bande (48) étant adaptée à la forme de l'élément de sécurité du tenon droit (46), **en ce que**, à proximité des éléments de fixation (08) sur la gouttière mandibulaire miniplast (03), sont prévus des éléments de recouvrement à surface lisse (20) qui dépassent dans la région molaire (09) de la gouttière mandibulaire miniplast (03) et recouvrent latéralement au moins partiellement les éléments de fixation (08), et/ou **en ce que** des marquages sont apposés sur les deux gouttières miniplast (34, 35) indiquant la position des deux gouttières miniplast (34, 35) l'une par rapport à l'autre.
